(19) Europäisches Patentamt
European Patent Office
Office européen des brevets

(11) EP 2 696 862 B1

(12) # FASCICULE DE BREVET EUROPEEN

(45) Date de publication et mention de la délivrance du brevet:
**15.03.2017 Bulletin 2017/11**

(21) Numéro de dépôt: **12722399.8**

(22) Date de dépôt: **11.04.2012**

(51) Int Cl.:
*A61K 31/155* (2006.01)
*A61K 31/7036* (2006.01)
*A61K 31/546* (2006.01)
*A61K 31/427* (2006.01)
*A61K 31/04* (2006.01)
*A61K 31/665* (2006.01)
*A61K 31/496* (2006.01)
*A61K 31/4188* (2006.01)
*A61K 45/06* (2006.01)
*A61K 31/57* (2006.01)
*A61K 31/43* (2006.01)
*A61K 31/7016* (2006.01)

(86) Numéro de dépôt international:
**PCT/FR2012/050789**

(87) Numéro de publication internationale:
**WO 2012/140364 (18.10.2012 Gazette 2012/42)**

(54) **UTILISATION DE CALIXARENES DANS LE TRAITEMENT DES INFECTIONS BACTERIENNES**
VERWENDUNG VON CALIXARENEN BEI DER BEHANDLUNG BAKTERIELLER INFEKTIONEN
USE OF CALIXARENES IN THE TREATMENT OF BACTERIAL INFECTIONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.04.2011 FR 1153204**

(43) Date de publication de la demande:
**19.02.2014 Bulletin 2014/08**

(73) Titulaire: **Université de Lorraine**
**54052 Nancy Cedex (FR)**

(72) Inventeurs:
- **GRARE, Marion**
**F-31300 Toulouse (FR)**
- **DUVAL, Raphaël, Emmanuel**
**F-57170 Bioncourt (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A2-95/19974 WO-A2-2006/042104**

- **GRARE MARION ET AL: "In vitro activity of para-guanidinoethylcalix[4] arene against susceptible and antibiotic-resistant Gram-negative and Gram-positive bacteria", JOURNAL OF ANTIMICROBIAL CHEMOTHERAPY, vol. 60, no. 3, septembre 2007 (2007-09), pages 575-581, XP002655308, ISSN: 0305-7453 cité dans la demande**
- **GRARE M ET AL: "Cationic compounds with activity against multidrug-resistant bacteria: interest of a new compound compared with two older antiseptics, hexamidine and chlorhexidine.", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES MAY 2010 LNKD- PUBMED:19456831, vol. 16, no. 5, mai 2010 (2010-05), pages 432-438, XP002655309, ISSN: 1469-0691 cité dans la demande**
- **GRARE M ET AL: "Cinetique d@?action du para-guanidinoethylcalix[4]arene, et evolution de la permeabilite membranaire", PATHOLOGIE ET BIOLOGIE, L'EXPANSION SCIENTIFIQUE FRANCAISE, PARIS, FR, vol. 58, no. 1, 1 février 2010 (2010-02-01), pages 46-51, XP026894782, ISSN: 0369-8114 [extrait le 2009-11-04] cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **MOURER M ET AL: "Functional organisation and gain of activity: The case of the antibacterial tetra-para-guanidinoethyl-calix[4]arene", BIOORGANIC & MEDICINAL CHEMISTRY LETTERS, PERGAMON, ELSEVIER SCIENCE, GB, vol. 16, no. 11, 1 juin 2006 (2006-06-01), pages 2960-2963, XP025106156, ISSN: 0960-894X, DOI: DOI:10.1016/J.BMCL.2006.02.072 [extrait le 2006-06-01] cité dans la demande**

**Description**

La présente invention concerne l'utilisation de calixarènes dans le traitement des infections bactériennes.

Dans le domaine de la santé publique, la lutte contre les infections bactériennes communautaires ou nosocomiales, est toujours un sujet d'actualité et d'inquiétudes. En effet, les bactéries sont les microorganismes le plus souvent responsables d'infections nosocomiales (IN), avec, par ordre de fréquence : *Escherichia coli* (24,7%), *Staphylococcus aureus* (18,9%), *Pseudomonas aeruginosa* (10%) et *Enterococcus spp.* (6%) (Enquête RAISIN 2006).

Certaines bactéries impliquées en clinique présentent une résistance voire, une multi-résistance aux antibiotiques et/ou antiseptiques utilisés en routine. On parle de BMR pour Bactéries Multi-Résistantes et de BTR pour Bactéries Toto-Résistantes. On peut citer par exemple les SARM *(Staphylococcus aureus* Résistants à la Méticilline, avec une résistance à l'ensemble des β-Lactamines), Entérobactéries porteuses de BLSE (β-Lactamase à Spectre Etendu) ou encore les ERG *(Enterococcus* spp. Résistants aux Glycopeptides). A l'heure actuelle, 64% des *Staphylococcus aureus* isolés au cours d'IN sont résistants à la méticilline (Enquête RAISIN 2006). Le problème est que les bactéries sont souvent porteuses de plusieurs mécanismes de résistance, induisant une résistance à de nombreuses familles d'antibiotiques: β-lactamines, aminosides, fluoroquinolones ou macrolides... Par ailleurs, cette résistance aux antibiotiques, est souvent associée à une résistance aux antiseptiques, utilisés en milieu hospitalier, pour lutter contre la dissémination des infections nosocomiales.

La résistance d'une souche bactérienne vis-à-vis d'un antibiotique peut être une résistance naturelle (caractéristique de l'ensemble des souches d'une même espèce). Elle peut être aussi acquise (caractéristique de certaines souches au sein d'une espèce); elle résulte alors d'une modification du capital génétique de ces bactéries. Ce genre de modification génétique peut conférer à une souche bactérienne concernée un mécanisme de résistance à un antibiotique, à une famille d'antibiotiques ou à plusieurs familles d'antibiotiques.

La recherche fondamentale sur les mécanismes mis en jeu par les bactéries et les informations épidémiologiques suscitent actuellement des doutes sur la possibilité d'éradiquer ces BMR dans l'avenir. Dès lors, il n'est plus certain que les antibiotiques disponibles actuellement permettent de contrôler durablement le problème. Si la mise à disposition de nouveaux antibiotiques a permis jusqu'à aujourd'hui de répondre à chaque forme de résistance bactérienne, cette démarche fait maintenant face à de nombreuses limites, puisque aucune nouvelle classe d'antibiotiques n'a été développée depuis vingt-cinq ans (Boucher et al. CID, 2009). Peu de nouveaux antibiotiques ont été commercialisés depuis le début des années 90. Parmi ces nouveaux antibiotiques, seuls le linézolide et la daptomycine, ont un mécanisme d'action innovant, mais sont réservés à des applications bien particulières et actifs uniquement sur les bactéries à Gram positif. En outre, ils présentent une toxicité importante (toxicité hématologique et médullaire pour le linézolide, pneumopathies à éosinophiles pour la daptomycine), ce qui a restreint leur usage.

Cependant, très peu de temps après leur commercialisation, des résistances bactériennes apparaissent. Ainsi, à titre d'exemple, on peut citer les cas du linézolide, de la daptomycine, de la quinupristine-dalfopristine, ou de la tigécycline, y compris chez des bactéries initialement BMR.

En mettant en jeu un concept innovant liant chimie supramoléculaire avec ciblage et désorganisation de la paroi bactérienne, une nouvelle famille de composés antibactériens, en particulier le para-guanidinoéthylcalix[4]arène, désigné ci-après Cx1, a été développée récemment. Cette famille de composés présente des propriétés antibactériennes vis-à-vis de différentes bactéries impliquées dans les infections nosocomiales et/ou communautaires.

La publication de Grare et al. (J. Antimicrob. Chemother. 60 (2007), 575-581) décrit que le Cx1 possède une activité antibactérienne sur les bactéries résistantes ou non aux antibiotiques.

Dans la publication de Grare et al. (Clin. Microbiol. Infect. 16 (2010), 432-438), l'activité antibactérienne du Cx1 est comparée à celle de l'hexamidine et de la chlorhexidine, deux antiseptiques, d'utilisation très courante en thérapeutique humaine, sur toute une série d'isolats cliniques : MDR (« multidrug resistant »), XDR (« extended drug resistant »), voire PDR (« pan-drug resistant »).

L'article de Grare et al. (Pathologie Biologie 58 (2010), 46-51) décrit que le Cx1, en tant qu'antibactérien cationique, interagit avec la paroi bactérienne, entraînant au final une perte d'intégrité membranaire.

Néanmoins, face à l'émergence et à la dissémination de nombreuses BMR, l'urgence absolue reste de pouvoir disposer, de nouveaux composés antibactériens possédant des mécanismes d'action innovants pour traiter les patients infectés par ce type de bactéries ; et/ou de moyens nouveaux permettant de rendre le traitement par des antibiotiques utilisés normalement en thérapeutique anti-infectieuse, à nouveau accessibles à ces patients.

Un des aspects de l'invention est de fournir de nouveaux composés permettant de rendre les antibiotiques à nouveau accessibles aux patients présentant des infections à BMR.

Le premier aspect de l'invention concerne un produit comprenant un calixarène représenté par la formule I suivante :

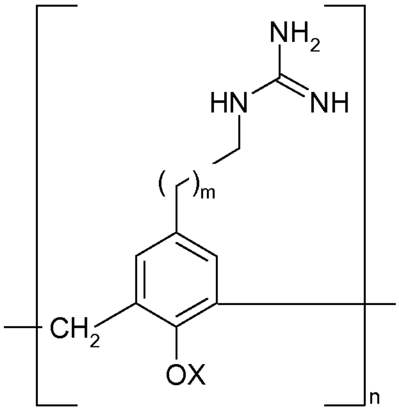

Formule I

,

dans lequel :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation dans le traitement de pathologies impliquant au moins une souche bactérienne présentant une résistance à au moins un « antibiotique déterminé », sur des patients sous traitement simultané ou séquentiel par un « antibiotique donné » vis-à-vis duquel ladite souche bactérienne présente éventuellement une résistance, ou
pour son utilisation dans le traitement de pathologies impliquant une souche bactérienne de phénotype sauvage ne présentant de résistance acquise à aucun antibiotique connu, sur des patients sous traitement simultané ou séquentiel par un « antibiotique donné » vis-à-vis duquel ladite souche bactérienne ne présente pas de résistance.

Dans un mode de réalisation, l'invention concerne un produit comprenant un calixarène représenté par la formule I suivante :

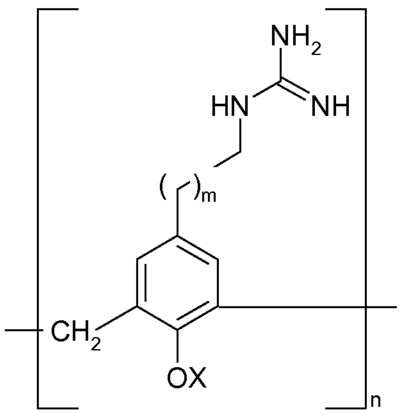

Formule I

,

dans lequel :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation dans le traitement de pathologies impliquant au moins une souche bactérienne présentant une résistance à au moins un « antibiotique déterminé », sur des patients sous traitement simultané ou séquentiel par un « antibiotique donné » vis-à-vis duquel ladite souche bactérienne présente éventuellement une résistance.

La présente invention propose un produit représenté par la formule I, qui permet de viser une catégorie spécifique et nouvelle de patients, qui, sous traitement d'un antibiotique désigné « antibiotique donné », présentent une infection à au moins une souche bactérienne de phénotype sauvage ou résistante à un ou plusieurs antibiotiques désignés « antibiotiques déterminés ».

On entend par « une souche bactérienne de phénotype sauvage » une souche bactérienne qui ne possède pas de mécanismes de résistance acquise aux antibiotiques (uniquement des résistances naturelles).

On entend par « une souche bactérienne présentant une résistance » une souche bactérienne présentant une résistance acquise vis-à-vis de laquelle la CMI (Concentration minimale Inhibitrice) de l'antibiotique testé est supérieure à la concentration critique établie par EUCAST (European Committee on Antimicrobial Susceptibility Testing).

On entend par « résistance naturelle » une caractéristique propre à toutes les souches d'une même espèce bactérienne. On entend par « résistance acquise » une caractéristique de certaines souches au sein d'une même espèce bactérienne.

La résistance acquise peut résulter d'une modification du capital génétique permettant à une souche bactérienne de tolérer une concentration d'antibiotique plus élevée que celle qui inhibe les souches sensibles de la même espèce.

Ladite résistance acquise peut être une résistance chromosomique résultant d'une mutation suivi par une sélection exercée par un antibiotique.

Cette résistance acquise peut être également une résistance extra-chromosomique résultant d'un transfert d'informations génétiques exogènes par plasmide, transposon ou intégron.

Du fait de l'existence de cette résistance, les antibiotiques sont administrés à des doses très élevées aux patients, ou parfois même ne sont plus actifs et ne peuvent plus être utilisés.

La présente invention repose sur un fait inattendu constaté par les Inventeurs, lors de l'évaluation de l'activité antibactérienne du para-guanidinoéthylcalix[4]arène, désigné ci-après Cx1. Cette molécule permet de diminuer la CMI d'un antibiotique vis-à-vis duquel une souche bactérienne présente une résistance.

En d'autre terme, d'un côté, le Cx1 permet de conférer *de novo* un certain niveau de sensibilité vis-à-vis d'(un) antibiotique(s) chez une souche bactérienne présentant une résistance acquise audit(s) antibiotique(s), et d'un autre côté, le Cx1 est aussi capable de conférer une sensibilité vis-à-vis d'(un) antibiotique(s) chez une souche bactérienne présentant une résistance naturelle audit(s) antibiotique(s).

Concrètement, dans le cadre clinique, le traitement par du Cx1 en association avec au moins un antibiotique permet de diminuer la dose de ce dernier dans le cadre du traitement d'une infection par une bactérie résistante audit antibiotique, et/ou de rendre le traitement par ledit antibiotique efficace chez les patients présentant une infection à au moins une souche bactérienne résistante audit antibiotique.

Le calixarène selon l'invention peut être administré à un patient simultanément ou séquentiellement avec un antibiotique désigné « antibiotique donné », ledit patient ayant une pathologie impliquant une souche bactérienne présentant une résistance à au moins un antibiotique désigné antibiotique déterminé.

Ladite souche peut présenter une résistance à plusieurs « antibiotiques déterminés ».

La dite souche peut présenter ou non une résistance à un « antibiotique donné ».

Par « antibiotique déterminé », on entend un antibiotique vis-à-vis duquel une souche bactérienne est résistante.

Par « antibiotique donné », on entend un antibiotique mis en oeuvre dans un traitement thérapeutique. Un patient sous traitement d'un « antibiotique donné » peut présenter ou non une résistance vis-à-vis de cet antibiotique.

Ladite souche peut présenter une résistance à plusieurs « antibiotiques donnés ».

Un « antibiotique déterminé » ou un « antibiotique donné » est un antibiotique connu de l'homme du métier pour son activité antibactérienne sur certaines souches de bactéries.

Ledit « antibiotique déterminé » ou « antibiotique donné » est différent du calixarène représenté par la formule I.

On entend par « patients sous traitement simultané (...) par un antibiotique donné », des patients qui reçoivent parallèlement au moins pendant une certaine période, un traitement par un antibiotique désigné « antibiotique donné », et un traitement par un calixarène selon l'invention. Par ailleurs, les deux susdits traitements peuvent être initialisés ou terminés l'un après l'autre.

La période de traitement simultané par un antibiotique et par un calixarène, déterminée selon l'état de santé

du patient, peut varier entre 5 jours et 4 semaines.

Lorsque le patient est sous traitement simultané d'un antibiotique et d'un calixarène selon l'invention, l'administration dudit antibiotique classique et celle du calixarène peuvent être effectuées au même moment ou l'une après l'autre selon un cycle de répétition fixé, par exemple l'une après l'autre dans un intervalle du temps adéquat.

On entend par « patients sous traitement séquentiel par un antibiotique donné », des patients qui reçoivent un traitement par un antibiotique avant ou après un traitement par un calixarène selon l'invention. Autrement dit, l'un des deux traitements poursuit l'autre immédiatement après l'achèvement de ce dernier, ou après une période adéquate de repos, par exemple entre 1 jour et une semaine.

Dans un mode de réalisation particulier, la souche bactérienne infectant le patient présente une résistance acquise à au moins un antibiotique déterminé.

Dans un mode de réalisation plus particulier, la souche bactérienne infectant le patient présente une résistance acquise à au moins deux « antibiotiques déterminés ».

Ces deux « antibiotiques déterminés » peuvent appartenir à la même famille d'antibiotiques ou à des familles différentes.

« L'antibiotique donné », administré au patient, peut être un antibiotique identique à ou différent d'un antibiotique déterminé, vis-à-vis duquel la souche bactérienne présente une résistance acquise.

La souche bactérienne infectant le patient peut être sensible à « l'antibiotique donné », ou présenter une résistance acquise ou naturelle.

Dans un mode de réalisation particulier, « l'antibiotique donné » administré au patient est différent de « l'antibiotique déterminé ». La souche bactérienne infectant le patient présente une résistance acquise à la fois à au moins un « antibiotique déterminé », et à un « antibiotique donné » administré audit patient.

Dans ce cas, le produit de formule I selon l'invention peut être utilisé dans le traitement de pathologies impliquant au moins une souche bactérienne présentant une résistance acquise à au moins un « antibiotique déterminé », sur des patients sous traitement simultané ou séquentiel par un antibiotique donné vis-à-vis duquel ladite souche bactérienne présente une résistance acquise.

Dans un mode de réalisation plus particulier, la souche bactérienne infectant le patient présente une résistance acquise à la fois à au moins deux « antibiotiques déterminés », et à un « antibiotique donné » administré audit patient.

Au cours d'un traitement par un antibiotique (antibiotique donné) pour une pathologie impliquant une souche bactérienne connue pour sa résistance acquise à au moins un autre antibiotique (« antibiotique déterminé »), si ladite souche bactérienne, responsable de l'infection acquiert une résistance audit « antibiotique donné », l'administration simultanée ou séquentielle d'un calixarène selon l'invention permet soit de restaurer la sensibilité de ladite souche audit « antibiotique donné », soit de diminuer la dose de « l'antibiotique donné » administré au patient.

Dans un autre mode de réalisation très particulier, « l'antibiotique donné », administré au patient, fait partie des antibiotiques déterminés, vis-à-vis desquels la souche bactérienne présente une résistance.

Dans ce cas, lorsqu'un patient présente une pathologie impliquant une souche bactérienne connue pour une résistance acquise à la fois à un « antibiotique déterminé » et à « un antibiotique donné », l'administration simultanée ou séquentielle d'un produit selon l'invention et de « l'antibiotique donné » permet soit de diminuer la dose de ce dernier administrée au patient, soit de restaurer la sensibilité vis-à-vis de l'antibiotique donné.

Dans un autre mode de réalisation particulier selon l'invention, « l'antibiotique donné », administré au patient, est « l'antibiotique déterminé », vis-à-vis duquel la souche bactérienne présente une résistance.

Dans ce cas, le produit de formule I selon l'invention peut être utilisé dans le traitement de pathologies impliquant une souche bactérienne présentant une résistance acquise à au moins un « antibiotique déterminé », sur des patients sous traitement simultané ou séquentiel par ledit antibiotique.

A titre d'exemple, si au cours d'un traitement par un antibiotique donné, ladite souche acquiert une résistance audit antibiotique, l'administration simultanée ou séquentielle d'un calixarène de formule I selon l'invention permet de restaurer la sensibilité audit « antibiotique donné ».

Aussi à titre d'exemple, lorsqu'un patient présente une pathologie impliquant une souche bactérienne connue pour sa résistance acquise à un « antibiotique donné », le traitement simultané ou séquentiel du produit de formule I selon l'invention et dudit « antibiotique donné » permet soit de diminuer la dose de ce dernier administrée au patient, soit de restaurer la sensibilité de ladite souche bactérienne vis-à-vis de l'antibiotique donné.

Dans un autre mode de réalisation particulier, la souche bactérienne infectant le patient est sensible à un « antibiotique donné » administré audit patient, mais ladite souche présente une résistance acquise à au moins un « antibiotique déterminé ».

Dans ce cas, le produit de formule I selon l'invention peut être utilisé dans le traitement de pathologies impliquant au moins une souche bactérienne présentant une résistance acquise à au moins un « antibiotique déterminé », sur des patients sous traitement simultané ou séquentiel par un « antibiotique donné » vis-à-vis duquel ladite souche bactérienne est sensible.

Lorsqu'un patient présente une pathologie impliquant une souche bactérienne connue pour sa résistance

acquise à au moins un « antibiotique déterminé », l'administration simultanée ou séquentielle du produit de formule I selon l'invention et d'un autre antibiotique (« antibiotique donné ») vis-à-vis duquel ladite souche bactérienne est sensible, permet de diminuer la dose dudit « antibiotique donné » administrée au patient.

Dans un mode de réalisation plus particulier, la souche bactérienne infectant le patient qui est sensible à un « antibiotique donné » administré audit patient, présente une résistance acquise à au moins deux « antibiotiques déterminés ».

Dans un autre mode de réalisation particulier, la souche bactérienne infectant le patient présente une résistance naturelle à un « antibiotique donné » administré audit patient, et une résistance acquise à au moins un « antibiotique déterminé ».

Le produit de formule I selon l'invention peut être utilisé dans le traitement de pathologie impliquant au moins une souche bactérienne présentant une résistance acquise à au moins un « antibiotique déterminé », sur des patients sous traitement simultané ou séquentiel par un « antibiotique donné » vis-à-vis duquel ladite souche bactérienne présente une résistance naturelle.

Dans cette situation, l'administration simultanée ou séquentielle d'un produit de formule I selon l'invention et d'un « antibiotique donné », permet de conférer une sensibilité à ladite souche bactérienne vis-à-vis dudit « antibiotique donné », et par conséquent de diminuer la dose de « l'antibiotique donné » administré au patient.

Dans un mode de réalisation plus particulier, la souche bactérienne infectant le patient qui présente une résistance naturelle à un « antibiotique donné » administré audit patient, présente une résistance acquise à au moins deux « antibiotiques déterminés ».

Dans un autre mode de réalisation, la souche bactérienne infectant le patient présente une résistance naturelle à au moins un « antibiotique déterminé ».

« L'antibiotique donné », administré au patient, peut être un antibiotique identique à ou différent d'un « antibiotique déterminé », vis-à-vis duquel la souche bactérienne présente une résistance naturelle.

La souche bactérienne infectant le patient peut être sensible à « l'antibiotique donné », ou présenter une résistance acquise ou naturelle.

Dans un autre mode de réalisation, la souche bactérienne infectant le patient présente une résistance naturelle à au moins deux « antibiotiques déterminés ».

Dans un mode de réalisation particulier, la souche bactérienne infectant le patient présente une résistance naturelle à un « antibiotique donné » administré audit patient, et à au moins un « antibiotique déterminé » qui est différent de « l'antibiotique donné ».

Le produit de formule I selon l'invention peut être utilisé dans le traitement de pathologies impliquant au moins une souche bactérienne présentant une résistance naturelle à au moins un « antibiotique déterminé », sur des patients sous traitement simultané ou séquentiel par un « antibiotique donné » vis-à-vis duquel ladite souche bactérienne présente une résistance naturelle.

L'administration simultanée ou séquentielle du produit de formule I selon l'invention et d'un « antibiotique donné », permet de conférer à ladite souche une sensibilité à « l'antibiotique donné » administré au patient et par conséquent de diminuer la dose de « l'antibiotique donné » administré au patient.

Dans un mode de réalisation plus particulier, la souche bactérienne infectant le patient présente une résistance naturelle à un « antibiotique donné » administré audit patient, et à au moins deux « antibiotiques déterminés » qui sont différents de l'antibiotique donné.

Dans un autre mode de réalisation particulier, la souche bactérienne infectant le patient présente une résistance naturelle à au moins un « antibiotique déterminé », qui est identique à « l'antibiotique donné » administré au patient.

Le produit de formule I selon l'invention peut être utilisé dans le traitement de pathologies impliquant une souche bactérienne présentant une résistance naturelle à au moins un « antibiotique déterminé », sur des patients sous traitement simultané ou séquentiel par ledit antibiotique.

L'administration simultanée ou séquentielle du produit de formule I selon l'invention et d'un « antibiotique donné », permet de conférer à ladite souche une sensibilité à « l'antibiotique donné » administré au patient et par conséquent de diminuer la dose de « l'antibiotique donné » administré au patient.

Dans un mode de réalisation plus particulier, la souche bactérienne infectant le patient présente une résistance naturelle à au moins deux « antibiotiques déterminés », dont l'un deux est identique à « l'antibiotique donné » administré au patient.

Dans un autre mode de réalisation particulier, la souche bactérienne infectant le patient présente une résistance naturelle à au moins un « antibiotique déterminé », et est sensible à « l'antibiotique donné » administré audit patient.

Le produit de formule I selon l'invention peut être utilisé dans le traitement de pathologies impliquant au moins une souche bactérienne présentant une résistance naturelle à au moins un « antibiotique déterminé », sur des patients sous traitement simultané ou séquentiel par un « antibiotique donné » vis-à-vis duquel ladite souche bactérienne est sensible.

L'administration simultanée ou séquentielle du produit de formule I selon l'invention et d'un « antibiotique

donné », permet de diminuer la dose de « l'antibiotique donné » administré audit patient.

Dans un mode de réalisation particulier, la souche bactérienne infectant le patient présente une résistance naturelle à au moins deux « antibiotiques déterminés », et est sensible à « l'antibiotique donné » administré audit patient.

Dans un autre mode de réalisation particulier, la souche bactérienne infectant le patient présente une résistance naturelle à au moins un « antibiotique déterminé », et une résistance acquise à « l'antibiotique donné » administré audit patient.

Le produit de formule I selon l'invention peut être utilisé dans le traitement de pathologies impliquant au moins une souche bactérienne présentant une résistance naturelle à au moins un « antibiotique déterminé », sur des patients sous traitement simultané ou séquentiel par un « antibiotique donné » vis-à-vis duquel ladite souche bactérienne présente une résistance acquise.

L'administration simultanée ou séquentielle du produit de formule I selon l'invention et d'un « antibiotique donné », permet soit de restaurer une sensibilité de ladite souche bactérienne vis-à-vis de « l'antibiotique donné », soit de diminuer la dose de l'antibiotique donné administré au patient.

Dans un mode de réalisation particulier, la souche bactérienne infectant le patient présente une résistance naturelle à au moins deux « antibiotiques déterminés », et une résistance acquise à « l'antibiotique donné » administré audit patient.

Dans un autre mode de réalisation, l'invention concerne un produit comprenant un calixarène représenté par la formule I suivante :

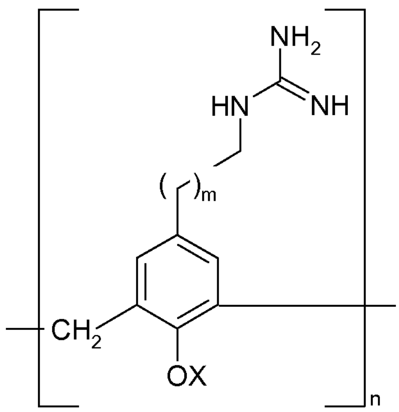

Formule I

,

dans lequel :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation dans le traitement de pathologies impliquant une souche bactérienne de phénotype sauvage ne présentant de résistance acquise à aucun antibiotique, sur des patients sous traitement simultané ou séquentiel par un « antibiotique donné » vis-à-vis duquel ladite souche bactérienne ne présente pas de résistance.

L'administration du produit de formule I selon l'invention simultanément ou séquentiellement avec l'administration d'un « antibiotique donné » permet de diminuer la dose de « l'antibiotique donné » administré.

Selon un mode de réalisation avantageux de l'invention, le calixarène est un macrocycle (oligomère cyclique) constitué de n unités phénoliques para-substituées ou non et reliées entre elles par des ponts méthyléniques.

Dans un mode de réalisation avantageux de l'invention, le calixarène impliqué est constitué de 4 unités, ledit calixarène correspondant à la formule I(1) suivante :

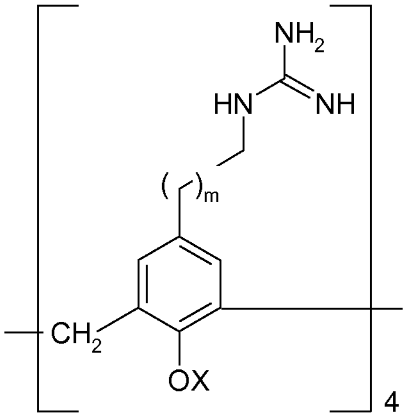

Formule I (1)

m et X ayant les significations indiquées ci-dessus.

Dans un autre mode de réalisation avantageux de l'invention, le calixarène impliqué est représenté par la formule I, dans laquelle m=1, ledit calixarène correspondant à la formule I(2) suivante :

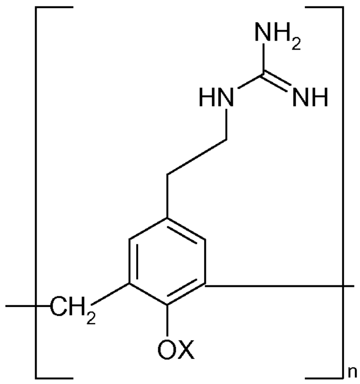

Formule I (2)

n et X ayant les significations indiquées ci-dessus.

Dans un autre mode de réalisation avantageux de l'invention, le calixarène impliqué est représenté par la formule I, dans laquelle X est un hydrogène, ledit calixarène correspondant à la formule I(3) suivante :

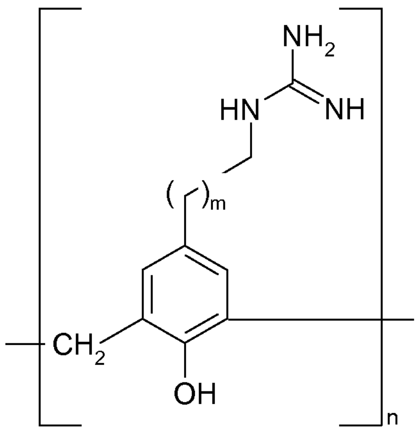

Formule I (3)

m et n ayant les significations indiquées ci-dessus.

Un mode de réalisation particulièrement avantageux de l'invention concerne un produit pour les utilisations décrites ci-dessus, ledit produit comprend un calixarène représenté par la formule I, dans laquelle : n=4, m=1, X est un hydrogène, ledit calixarène correspondant à la formule II suivante :

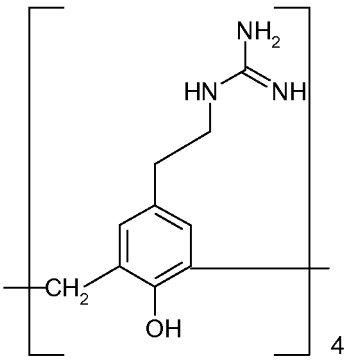

Formule II

La molécule représentée par la formule II est le para-guanidinoéthylcalix[4]arène, désigné par Cx1 dans la présente invention.

La structure tridimensionnelle de la susdite molécule est illustrée ci-après.

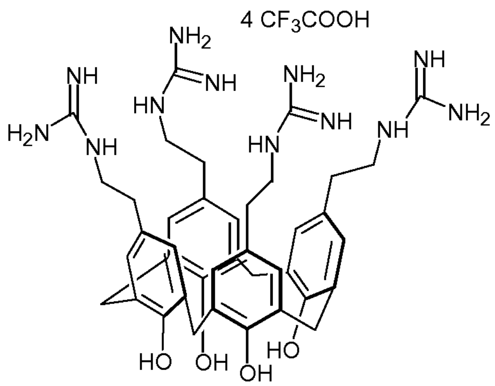

Le Cx1 peut être synthétisé selon le procédé décrit dans Mourer et al. (Bioorganic & Médicinal Chemistry Letter 16 (2006) 2960-2963).

Le calixarène impliqué dans l'invention peut être tel que décrit ci-dessus, ou un sel d'acide physiologiquement acceptable dérivé d'un composé de formule (I) tel qu'un chlorhydrate, un formiate, un trifluoroacétate ou un oxalate (HOOCCOOH).

L'expression « sel d'acide physiologiquement acceptable» signifie un dérivé d'un composé de formule I, obtenu par réaction d'un acide inorganique ou d'un acide organique, sur un composé de formule I.

Des exemples d'acides inorganiques permettant l'obtention de sels physiologiquement acceptables incluent sans être limités à ceux-ci, l'acide chlorhydrique, l'acide bromhydrique, l'acide nitrique, l'acide carbonique, l'acide formique, l'acide monohydrogénocarbonique, l'acide phosphorique, l'acide monohydrogénophosphorique, l'acide dihydrogéno-phosphorique, l'acide perchlorique, l'acide sulfurique, l'acide monohydrogénosulfurique, l'acide iodhydrique.

Des exemples d'acides organiques permettant l'obtention de sels physiologiquement acceptables incluent sans être limités à ceux-ci, l'acide acétique, l'acide lactique, l'acide propionique, l'acide butyrique, l'acide isobutyrique, l'acide palmique, l'acide maléique, l'acide glutamique, l'acide hydroxymaléique, l'acide malonique, l'acide benzoïque, l'acide succinique, l'acide glycolique, l'acide subérique, l'acide fumarique, l'acide mandélique, l'acide phthalique, l'acide salicylique, l'acide benzènesulfonique, l'acide *p*-toluènesulfonique, l'acide citrique, l'acide tartrique, l'acide méthanesulfonique, l'acide hydroxynaphthoïque.

Les sels d'acides aminés, tels que les arginates et leurs équivalents sont également inclus ainsi que les sels d'acides organiques tels que l'acide glucuronique ou l'acide galacturonique et leurs équivalents (voir, par exemple, Berge et al, "Pharmaceutical Salts", Journal of Pharmaceutical Science, 1977, 66, 1-19).

Le calixarène impliqué dans l'invention peut être utilisé dans le traitement des pathologies impliquant une souche bactérienne présentant une résistance, à au moins une famille d'antibiotiques, notamment dans le traitement des infections nosocomiales et/ou communautaires, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéoarticulaires, les infections oculaires, les septicémies ou bactériémies.

Le tableau 1 ci-dessous décrit plus en détail les infections pouvant être traitées par un calixarène de formule

I selon l'invention.

| | |
|---|---|
| infections abdominales | péritonite, appendicite... |
| infections digestives | diarrhées toxi-infection alimentaire collectives, diarrhées post-antibiothérapie... |
| infections urinaires | cystites, pyélonéphrites, prostatites... |
| infections respiratoires | bronchites, pneumonies, pneumopathies, abcès... |
| infections neuro-méningées | méningites bactériennes, abcès cérébraux... |
| infections de la sphère oropharyngée | sinusites, otites, angines, phlegmons, épiglottites... |
| infections génitales | vulvite, vaginite/vaginose, cervicite, salpingite... |
| infections de la peau et des tissus mous | furonculose, abcès, escarre, pied diabétique ... |
| infections oculaires | conjonctivites, kératites, endophtalmies... |
| autres infections | septicémies ou bactériémies... |

Plus particulièrement, le calixarène impliqué dans l'invention peut être utilisé dans le traitement des pathologies impliquant une souche bactérienne résistante appartenant à une espèce choisie parmi *Escherichia coli, Pseudomonas aeruginosa* et *Staphylococcus* $\alpha$*ureus.*

Encore plus particulièrement, le calixarène impliqué dans l'invention peut être utilisé dans le traitement des pathologies impliquant une souche de bactéries résistantes choisies parmi :

- une souche de *Staphylococcus aureus* de phénotype sauvage
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergystines et à l'ofloxacine,
- une souche *d'Escherichia coli* de phénotype sauvage
- une souche *d'Escherichia coli* productrice de pénicillinase sans résistance associée,
- une souche *d'Escherichia coli* productrice de BLSE (β-Lactamase à Spectre Etendu), présentant une résistance associée aux aminosides, à la rifampicine et à l'association triméthoprime-sulfaméthoxazo le,
- une souche *d'Escherichia coli* hyperproductrice de céphalosporinase présentant une résistance associée aux aminosides, aux quinolones et à l'association triméthoprime-sulfaméthoxazole,
- une souche de *Pseudomonas aeruginosa* de phénotype sauvage
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénèmes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

On entend « une souche de *Staphylococcus aureus* de phénotype sauvage », une souche de *Staphylococcus aureus* qui ne possède pas de mécanisme de résistance acquise aux antibiotiques (uniquement des résistances naturelles).

On entend « une souche *d'Escherichia coli* de phénotype sauvage », une souche *d'E.coli* qui ne possède pas de mécanismes de résistance acquise aux antibiotiques (uniquement des résistances naturelles).

On entend « une souche de *Pseudomonas aeruginosa* de phénotype sauvage », une souche de *P. aeruginosa* qui ne possède pas de mécanisme de résistance acquise aux antibiotiques (uniquement des résistances naturelles).

Dans un mode de réalisation particulier, le calixarène de formule I selon l'invention peut être utilisé sur des patients sous traitement simultané ou séquentiel par un antibiotique donné choisi dans le groupe constitué des β-lactamines, des aminosides, des fluoroquinolones, de la fosfomycine, de la colimycine, de la rifampicine, de la tigécycline, ou de l'acide fusidique.

Les antibiotiques de la famille des β- lactamines comprennent tout antibiotique qui contient un noyau β-lactame

dans sa structure moléculaire, tels que l'amoxicilline, l'oxacilline, l'imipénème, la ticarcilline, la pipéracilline, l'aztréonam, la céfépime, la céfotaxime, et la ceftazidime.

La famille des aminosides comprend, entre autre : l'amikacine, la tobramycine, la streptomycine, ou la gentamicine.

La famille des ftuoroquinolones comprend les dérivés de l'acide nalidixique, tels que la norfloxacine, la ciprofloxacine, la lévofloxacine, ou la moxifloxacine.

Dans un mode de réalisation plus particulier de l'invention, l'antibiotique donné peut être choisi parmi : l'imipénème, la pipéracilline-tazobactam, la pénicilline G, le céfotaxime, la ceftazidime, la tobramycine, la gentamicine, la ciprofloxacine, la rifampicine, la fosfomycine, la colimycine, la tigécycline, la ticarcilline-acide clavulanique, la streptomycine ou l'acide fusidique.

A titre d'exemple, le calixarène de formule I selon l'invention peut être utilisé sur des patients atteints de pathologies impliquant une souche de *Staphylococcus aureus* de phénotype sauvage, sous traitement simultané ou séquentiel par un antibiotique donné choisi parmi la tigécycline, l'acide fusidique ou la fosfomycine.

Aussi, à titre d'exemple, le calixarène de formule I selon l'invention peut être utilisé sur des patients atteints de pathologies impliquant une souche de SARM sans résistance associée, sous traitement simultané ou séquentiel par un antibiotique donné choisi parmi la streptomycine, la tigécycline, l'acide fusidique, ou la fosfomycine.

Aussi, à titre d'exemple, le calixarène de formule I selon l'invention peut être utilisé sur des patients atteints de pathologies impliquant une souche de SARM présentant une résistance aux aminosides et aux fluoroquinolones, sous traitement simultané ou séquentiel par un antibiotique donné choisi parmi la pénicilline G, la tigécycline, l'acide fusidique ou la fosfomycine.

Aussi, à titre d'exemple, le calixarène de formule I selon l'invention peut être utilisé sur des patients atteints de pathologies impliquant une souche de SARM présentant une résistance aux aminosides, aux fluoroquinolones, aux macrolides-lincosamides-synergistines et à l'ofloxacine, sous traitement simultané ou séquentiel par un antibiotique donné choisi parmi la pénicilline G, l'imipénème, la gentamicine, la tigécycline, l'acide fusidique ou la fosfomycine.

Aussi, à titre d'exemple, le calixarène de formule I selon l'invention peut être utilisé sur des patients atteints de pathologies impliquant une souche *d'Escherichia coli* de phénotype sauvage, sous traitement simultané ou séquentiel par un antibiotique donné choisi parmi la gentamicine, la tobramycine, ou la rifampicine.

Aussi, à titre d'exemple, le calixarène de formule I selon l'invention peut être utilisé sur des patients atteints de pathologies impliquant une souche *d'Escherichia coli* productrice de BLSE présentant une résistance associée aux aminosides, à la rifampicine et à l'association triméthoprime-sulfaméthoxazole, sous traitement simultané ou séquentiel par un antibiotique donné choisi parmi le céfotaxime, la gentamicine ou la rifampicine.

Aussi, à titre d'exemple, le calixarène de formule I selon l'invention peut être utilisé sur des patients atteints de pathologies impliquant une souche *d'Escherichia coli* productrice de pénicillinase sans résistance associée, ou une souche *d'Escherichia coli* hyperproductrice de céphalosporinase présentant une résistance associée aux aminosides, aux quinolones et à l'association triméthoprime-sulfaméthoxazole, sous traitement simultané ou séquentiel par un antibiotique donné choisi parmi la gentamicine ou la rifampicine.

Aussi, à titre d'exemple, le calixarène de formule I selon l'invention peut être utilisé sur des patients atteints de pathologies impliquant une souche de *Pseudomonas aeruginosa* de phénotype sauvage, sous traitement simultané ou séquentiel par un antibiotique donné choisi parmi la pipéracilline-tazobactam, la ceftazidime, la tobramycine, la ciprofloxacine, la rifampicine, la fosfomycine ou la ticarcilline-acide clavulanique.

Aussi, à titre d'exemple, le calixarène de formule I selon l'invention peut être utilisé sur des patients atteints de pathologies impliquant une souche de *Pseudomonas aeruginosa* présentant une résistance aux β-lactamines, à l'association triméthoprime-sulfaméthoxazole et à la fosfomycine, sous traitement simultané ou séquentiel par un antibiotique donné choisi parmi la pipéracilline-tazobactam, la rifampicine, ou la tobramycine.

Aussi, à titre d'exemple, le calixarène de formule I selon l'invention peut être utilisé sur des patients atteints de pathologies impliquant une souche de *Pseudomonas aeruginosa* présentant une résistance aux β-lactamines (y compris les carbapénèmes), aux aminosides, à l'association triméthoprime-sulfaméthoxazole et à la ciprofloxacine, sous traitement simultané ou séquentiel par un antibiotique donné choisi parmi la ceftazidime, la rifampicine, la colimycine, la fosfomycine, ou la tobramycine.

Egalement, à titre d'exemple, le calixarène de formule I selon l'invention peut être utilisé sur des patients atteints de pathologies impliquant une souche muqueuse de *Pseudomonas aeruginosa* présentant une résistance à la rifampicine et à l'association triméthoprime-sulfaméthoxazole, sous traitement simultané ou séquentiel par un antibiotique donné choisi parmi la pipéracilline-tazobactam, l'imipénème, la rifampicine, la colimycine, la fosfomycine, la tobramycine ou la ciprofloxacine.

La présente demande décrit également une composition pharmaceutique comprenant au moins une composition telle que décrit ci-dessus comme substance active en association avec un véhicule pharmaceuticalement acceptable.

Diverses formulations sont possibles pour les dites compositions pharmaceutiques : sous forme de gélule,

comprimé, poudre, crème, lotion, solution aqueuse ou hydro-alcoolique, collutoire, collyre, lait, mousse, gel, spray ou poudre par exemple.

Ladite composition pharmaceutique peut être administrée par voie orale, parentérale, ou topique.

Dans une telle composition pharmaceutique selon l'invention, l'homme du métier sait que la dose d'administration unitaire du Cx1 dépend de la nature des bactéries à traiter, mais également de la dose unitaire d'un antibiotique donné. La dose d'administration unitaire d'un antibiotique donné classique est connue de l'homme du métier.

**Figures**

Figure 1 : la figure 1 représente une microplaque préparée pour mesurer la sensibilité d'une souche bactérienne vis-à-vis d'une solution contenant un antibiotique et le Cx1 respectivement dans une proportion des concentrations déterminées. Les colonnes 1 et 12 ne contiennent que du milieu témoin. Les colonnes 2 et 11 contiennent du milieu témoin et des bactéries, mais sans antibiotique ni le Cx1. Les colonnes 3 à 10 contiennent les bactéries, le Cx1 en concentration décroissante et un antibiotique en concentration croissante.
Figure 2A : la figure 2A illustre une additivité entre le Cx1 et un autre antibiotique.
Figure 2B : la figure 2B illustre une indifférence entre le Cx1 et un autre antibiotique.
Figure 2C : la figure 2C illustre une synergie entre le Cx1 et un autre antibiotique.
Figure 2D : la figure 2D illustre un antagonisme entre le Cx1 et un autre antibiotique.

**RESULTATS**

**1. Matériel et Méthode**

**1.1 Matériel et réactif**

- Seringue de 10, 20 ou 50 mL
- Filtre 0,22 μm (filtres Millex®GP, 0,22 μm, Millipore, France)
- Tubes Falcon 15 et 50 mL
- Plaques de 96 puits (Greiner, 650161)
- Géloses Mueller Hinton (MHA) (Difco, 225250)
- Bouillons Mueller Hinton (MHB) (Difco, 275730)
- Eau distillée stérile

Solution de la drogue à tester : Cx1 (M = 1221,11 g/mol) fournie par le Pr. Regnouf de Vains sous forme de poudre blanche, reprise en eau distillée stérile et filtrée sur 0,22 μm pour obtenir une solution stérile à 10-2 mol/L. Nous nous sommes procuré les antibiotiques commerciaux par les fabricants, sous forme de poudre stérile, prête à l'emploi.

**1.2 Souches bactériennes**

Trois souches de référence ont été utilisées, correspondant à celles étudiées pour les CMI et les CMB (Concentration Minimale Bactéricide) : *Escherichia coli* ATCC 25922, *Staphylococcus aureus* ATCC 29213, *Pseudomonas aeruginosa* ATCC 27853. Pour chacune de ces souches ont été choisis 3 isolats cliniques correspondant, présentant divers profils de résistance aux antibiotiques classiquement utilisés en routine :

- EcR1, EcR2, EcR3;
- SaR1, SaR3, SaR4;
- PaR2, PaR3, PaR5.

Les profils de sensibilité aux antibiotiques de ces isolats cliniques sont figurés dans la partie ci-après « Identification et antibiogramme ».
Les fluctuations relatives au type de résistance associée pour certaines souches sont dues à l'évolution des recommandations françaises (CA-SFM), européennes (EUCAST) et américaines (CLSI) en matière de catégorisation des souches bactériennes.

### 1.3. Mode opératoire : Technique de l'échiquier

**J-1 : Mise en culture des bactéries sur gélose MHA (Mueller Hinton Agar)**

Incuber 24 h à 35°C.

**J0 : Ensemencement d'un bouillon MHB (Mueller Hinton Broth)**

Prélever une colonie « moyenne » sur la gélose J-1 et ensemencer 5 mL de bouillon MHB.
Incuber 24 h à 35°C.

**J1 : Préparation des plaques de 96 puits**

Préparation de l'inoculum bactérien :

La pureté des souches est vérifiée par l'absence de contaminants sur la gélose MHA ensemencée parallèlement au bouillon, et par réalisation d'une coloration de Gram.
La suspension bactérienne est transférée dans un tube Falcon de 15 mL, centrifugée 10 min à 4500g puis le culot est remis en suspension dans 1 mL d'eau distillée stérile. Les dilutions adéquates sont ensuite réalisées afin d'obtenir un inoculum bactérien entre $5.10^5$ et $5.10^6$ UFC/mL.

Préparer les solutions : 1) Antibiotique (ATB) à tester, 2) Cx1

Les CMI des antibiotiques ont été déterminées préalablement pour chaque souche, par méthode de microdilution en milieu liquide (CLSI (Clinical and Laboratory Standards Institute), 2003].
Les dilutions adéquates sont réalisées afin d'obtenir une solution de concentration équivalente à 32 fois la CMI de l'ATB à tester, en milieu MH (Mueller-Hinton). Puis nous avons procédé à une série de dilutions d'ordre 2 en milieu MH afin d'obtenir les concentrations suivantes : 16, 8, 4, 2, 1, 0,5 et 0,25 fois la CMI (tubes Falcon de 15 mL). Le même mode opératoire est suivi pour le Cx1. Cela permet d'obtenir une gamme de concentration de 8 à 0,06 fois la CMI dans la microplaque pour les deux molécules (dilution au demi avec addition de la 2ème molécule, puis nouvelle dilution au ½ après ajout de la suspension bactérienne). On obtient ainsi 64 combinaisons ATB/Cx1.
Pour chaque plaque, 1 mL de solution de chaque dilution est nécessaire.

Préparation des microplaques

Le volume final contenu dans chacun des puits doit être de 100 µL. Deux témoins doivent être présents sur chaque plaque :

- colonne 1 et 12 : témoin milieu
- colonne 2 et 11 : témoin milieu + bactéries

*Distribution du milieu MH*

100 µL dans chacun des puits des colonnes 1 et 12.
50 µL dans chacun des puits des colonnes 2 et 11.

*Distribution de la gamme de dilution de l'antibiotique à tester et de la molécule d'intérêt*

Cx1 : 25 µL dans les puits des colonnes 11 à 3, en commençant par la plus faible concentration.
ATB : 25 µL dans les puits des lignes H à A (colonnes 3 à 11), en commençant par la plus faible concentration.

*Distribution de la suspension bactérienne*

50 µL dans chacun des puits des colonnes 2 à 11.

**J2 : Lecture de la turbidité à 540 nm.**

Les différents types d'interactions observés par la technique de l'échiquier sont présentés dans les figures 2A,

2B, 2C et 2D :

La valeur FIC (Fractional Inhibitory Concentration Index) est déterminée par la formule suivante :

$$FIC = FIC_A + FIC_B = \frac{CMI_A \text{ en assoc}}{CMI_A \text{ seul}} + \frac{CMI_B \text{ en assoc}}{CMI_B \text{ seul}}$$

Lorsque FIC ≤ 0,5, il y a un effet synergique entre l'antibiotique A et l'antibiotique B.
Lorsque 0,5 < FIC ≤ 1, il y a un effet additif entre l'antibiotique A et l'antibiotique B.
Lorsque 1 < FIC ≤ 4, il y a un effet indifférent entre l'antibiotique A et l'antibiotique B.
Lorsque FIC > 4, il y a un effet antagoniste entre l'antibiotique A et l'antibiotique B.
Pour chaque souche et chaque combinaison antibiotique/Cx1, les expériences ont été répétées au minimum 3 fois.

**2. Résultats & Discussion**

- Les résultats obtenus sont présentés sous forme de tableaux (tableaux I à XII), présentant de façon plus détaillée et par couple ATB/Cx1 : les CMI des composés utilisés seuls (CMI initiales) ;
- les gammes testées ;
- les FIC index obtenus lors des différentes expériences ;
- les concentrations optimales pour la synergie ;
- le facteur de dilution de Cx1 correspondant à la différence de dosage existant entre l'utilisation de Cx1 seul ou en association avec un antibiotique ;
- le facteur de dilution de l'antibiotique correspondant à la différence de dosage existant entre l'utilisation de l'antibiotique seul ou en association avec Cx1 ;
- la nature de l'interaction observée.

Les résultats ci-après montrent qu'aucun antagonisme entre le Cx1 et un antibiotique testé n'a été observé, quel que soit la souche et les associations testées.
Les résultats démontrent également que le traitement par du Cx1 en association avec le traitement par un antibiotique pour une pathologie impliquant une souche bactérienne présentant une résistance audit antibiotique permet :

- de conférer un certain niveau de sensibilité de la souche bactérienne vis-à-vis dudit antibiotique ;
- de diminuer la dose dudit antibiotique ainsi que la dose du Cx1 administrées.

Les résultats démontrent que le traitement par du Cx1 en association avec le traitement par un antibiotique donné pour une pathologie impliquant une souche bactérienne présentant une résistance à un antibiotique déterminé permet de diminuer la dose d'antibiotique donné ainsi que la dose de Cx1 administrées.
Ces résultats sont également valables pour le traitement par du Cx1 en association avec le traitement par un antibiotique donné pour une pathologie impliquant une souche bactérienne présentant une résistance à au moins deux familles d'antibiotiques différentes. Tel est le cas des souches SaR3, EcR2, SaR4, PaR2 et PaR3 testées.
*Pseudomonas aeruginosa* est la souche, parmi toutes les souches analysées, sur laquelle le plus grand nombre d'associations synergiques, avec des antibiotiques très variés (β-lactamines, aminosides, fluoroquinolones...), a été observé.
Par ailleurs, les antibiotiques pour lesquels une synergie entre lesquels et le Cx1 a été observée agissent au niveau :

- de la paroi : fosfomycine (sachant que le mécanisme d'action spécifique de la fosfomycine se traduit par une synergie d'action quasi-constante avec les autres antibiotiques actifs sur la paroi bactérienne) mais aussi avec les associations pipéracilline-tazobactam & ticarcilline-acide clavulanique, et la ceftazidime ;
- de la synthèse des protéines : tigécycline, gentamicine, tobramycine, streptomycine, acide fusidique ;
- de la synthèse des acides nucléiques : rifampicine, ciprofloxacine.

**2. Synergie de l'association du Cx1 avec des antibiotiques vis-à-vis de souches *d'Escherichia coli* résistantes ou non aux antibiotiques**

**2.1. Tableau I : Synergie vis-à-vis de la souche *d'Escherichia coli* ATCC 25922 (phénotype sauvage) (n=4)**

| Associations | CMI initiales (mg/L) | Gammes testées (mg/L) | FIC index | CMI optimales (mg/L) | Différence CMI Cx1 | Différence CMI ATB | Conclusion |
|---|---|---|---|---|---|---|---|
| **Cx1/Amoxicilline** | 4/4 | 32/32 | 1-4 | nd | nd | Nd | Indifférence |
| **Cx1/Amoxicilline-Acide clavulanique** | 4/4 | 16/256 | 0,53-1 | 0,125/2 | ↓x5 | ↓ x1 | Additivité |
| **Cx1/Pipéracilline** | 4/2 | 16/256 | 1-4 | nd | nd | nd | Indifférence |
| **Cx1/Céfotaxime** | 4/0,06 | 16/1 | 0,53-1 | 0,125/0,03 | ↓ x5 | ↓ x1 | Additivité |
| **Cx1/Ceftazidime** | 4/0,125 | 16/1 | 0,62-0,98 | 2/0,015 | ↓ x1 | ↓ x3 | Additivité |
| **CxI/Imipénème** | 4/0,125 | 16/1 | 1-4 | nd | nd | nd | Indifférence |
| **Cx1/Ertapénème** | 4/0,015 | 16/1 | 0,625-1 | 1/0,07 | ↓ x2 | ↓x1 | Additivité |
| **Cx1/Gentamicine** | 4/0,125 | 16/4 | 0,27-0,98 | 0,125/0,03 | ↓x5 | ↓x2 | Synergie |
| **Cx1/Amikacine** | 4/0,25 | 16/4 | 1-4 | nd | nd | nd | Indifférence |
| **Cx1/Tobramycine** | 4/0,25 | 16/4 | 0,365-1 | 0,5/0,06 | ↓x3 | ↓ x2 | Synergie |
| **Cx1/Ciprofloxacine** | 2/0,015 | 16/1 | 1-4 | nd | nd | nd | Indifférence |
| **Cx1/Rifampicine** | 4/4 | 16/16 | 0,16-1 | 0,5/0,125 | ↓ x3 | ↓x5 | Synergie |

**2.2. Tableau II : Synergie vis-à-vis d'une souche *d'Escherichia coli* productrice de pénicillinase sans résistance associée (ou EcR1) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/>256 | 32/256 | 1-4 | nd | nd | nd | Indifférence |
| **Cx1/Amoxicilline-Acide clavulanique** | 2/16 | 16/256 | 1-4 | nd | nd | nd | Indifférence |
| **Cx1/Pipéracilline** | 2/256 | 16/256 | 1-4 | nd | nd | nd | Indifférence |
| **Cx1/Céfotaxime** | 2/0,06 | 16/1 | 0,75-1 | 1/0,015 | ↓ x1 | ↓ x2 | Additivité |
| **Cx1/Ceftazidime** | 2/0,25 | 16/1 | 0,56-1 | 0,125/0,125 | ↓ x4 | ↓ x1 | Additivité |
| **Cxl/Imipénème** | 2/0,125 | 16/1 | 1-4 | nd | nd | nd | Indifférence |
| **Cx1/Ertapénème** | 2/0,015 | 16/1 | 0,75-1 | 0,5/0,07 | ↓ x2 | ↓ x1 | Additivité |
| **Cx1/Gentamicine** | 2/0,125 | 16/4 | 0,3-1 | 0,125/0,03 | ↓ x4 | ↓ x2 | Synergie |
| **Cx1/Amikacine** | 2/0,25 | 16/4 | 1-4 | nd | nd | nd | Indifférence |
| **Cx1/Tobramycine** | 2/0,25 | 16/4 | 1-4 | nd | nd | nd | Indifférence |
| **Cx1/Ciprofloxacine** | 2/0,015 | 16/1 | 1-4 | nd | nd | nd | Indifférence |
| **Cx1/Rifampicine** | 2/4 | 16/16 | 0,28-1 | 0,5/0,125 | ↓ x2 | ↓ x5 | Synergie |

**2.3. Tableau III : Synergie vis-à-vis d'une souche de *Escherichia coli* productrice de BLSE présentant une résistance associée aux aminosides, à la rifampicine et à l'association triméthorpime-sulfaméthoxazole (ou EcR3) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/>256 | 16/256 | 1-4 | nd | nd | nd | Indifférence |
| **Cx1/Amoxicilline-Acide clavulanique** | 2/32 | 16/256 | 0,56-1 | 0,125/16 | x4 | x1 | Additivité |
| **Cx1/Pipéracilline** | 2/128 | 16/256 | 1-4 | nd | nd | nd | Indifférence |
| **Cx1/Céfotaxime** | 2/128 | 16/256 | 0,375-1 | 0,25/32 | ↓ x3 | ↓ x2 | Synergie |
| **Cx1/Ceftazidime** | 2/1 | 16/16 | 0,56-1 | 0,125/0,5 | ↓ x4 | ↓ x1 | Additivité |
| **Cx1/Imipénème** | 2/0,125 | 16/1 | 1-4 | nd | nd | nd | Indifférence |
| **Cx1/Ertapénème** | 2/0,0015 | 16/1 | 1-4 | nd | nd | nd | Indifférence |
| **Cx1/Gentamicine** | 2/2 | 16/4 | 0,31-1 | 0,125/0,5 | ↓ x4 | ↓ x3 | Synergie |
| **Cx1/Amikacine** | 2/1 | 16/4 | 1-4 | nd | nd | nd | Indifférence |
| **Cx1/Tobramycine** | 2/4 | 16/4 | 1-4 | nd | nd | nd | Indifférence |
| **Cx1/Ciprofloxacine** | 2/0,06 | 16/1 | 0,56-1 | 0,125/0,03 | ↓ x4 | ↓ x1 | Additivité |
| **Cx1/Rifampicine** | 2/4 | 16/16 | 0,25-1 | 0,25/0,5 | ↓ x3 | ↓ x3 | Synergie |

**2.4. Tableau IV : Synergie vis-à-vis d'une souche *d'Escherichia coli* hyperproductrice de céphalosporinase présentant une résistance associée aux aminosides, aux quinolones et à l'association triméthoprime-sulfaméthoxazole (ou EcR2) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Amoxicilline** | 2/256 | 16/256 | 1-4 | nd | nd | nd | Indifférence |
| **Cx1/Amoxicilline-Acide clavulanique** | 2/256 | 32/256 | 0,56-1 | 0,125/128 | ↓ x4 | ↓ x1 | Additivité |
| **Cx1/Pipéracilline** | 2/32 | 16/256 | 0,56-1 | 0,125/16 | ↓ x4 | ↓ x1 | Additivité |
| **Cx1/Céfotaxime** | 2/4 | 16/32 | 1-4 | nd | nd | nd | Indifférence |
| **Cx1/Ceftazidime** | 2/8 | 16/32 | 1-4 | nd | nd | nd | Indifférence |
| **Cx1/Imipénème** | 2/0,125 | 16/1 | 1-4 | nd | nd | nd | Indifférence |
| **Cx1/Ertapénème** | 2/0,03 | 16/1 | 1-4 | nd | nd | nd | Indifférence |
| **Cx1/Gentamicine** | 2/2 | 16/4 | 0,31-1 | 0,125/0,5 | ↓ x4 | ↓ x2 | Synergie |
| **Cx1/Amikacine** | 2/0,5 | 16/4 | 1-4 | nd | nd | nd | Indifférence |
| **Cx1/Tobramycine** | 2/4 | 16/4 | 1-4 | nd | nd | nd | Indifférence |
| **Cx1/Ciprofloxacine** | 2/0,015 | 16/1 | 1-4 | nd | nd | nd | Indifférence |
| **Cx1/Rifampicine** | 2/2 | 16/16 | 0,31-1 | 0,25/0,5 | ↓ x3 | ↓ x2 | Synergie |

**3. Synergie de l'association du Cx1 avec des antibiotiques vis-à-vis de souches de *Staphylococcus aureus* résistantes ou non aux antibiotiques**

**3.1. Tableau V : Synergie vis-à-vis de la souche *Staphylococcus aureus* ATCC 29213 (phénotype sauvage) (n=4)**

| Associations | CMI initiales (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/1 | 64/4 | 0,5-1 | 2/0,25 | ↓ x2 | ↓ x2 | Additivité |
| **Cxl/Imipénème** | 8/0,015 | 64/1 | 1-4 | nd | nd | nd | Indifférence |
| **Cx1/Erythromycine** | 8/0,5 | 64/4 | 1-4 | nd | nd | nd | Indifférence |
| **Cx1/Vancomycine** | 8/0,5 | 64/4 | 0,75-1 | 4/0,125 | ↓ x1 | ↓ x2 | Additivité |
| **Cx1/Lévofloxacine** | 8/0,125 | 64/4 | 1-4 | nd | nd | nd | Indifférence |
| **Cx1/Amikacine** | 8/1 | 64/4 | 0,53-1 | 4/0,03 | ↓ x1 | ↓ x5 | Additivité |
| **Cx1/Gentamicine** | 8/0,25 | 64/2 | 0,49-1 | 2/0,06 | ↓ x2 | ↓ x2 | Additivité |
| **Cx1/Streptomycine** | 8/4 | 64/32 | 0,375-1 | 1/1 | ↓ x3 | ↓ x2 | Synergie |
| **Cx1/Linézolide** | 8/2 | 64/8 | 1-4 | nd | nd | nd | Indifférence |
| **Cx1/Tigécycline** | 8/0,25 | 64/2 | 0,18-1 | 0,5/0,03 | ↓ x4 | ↓ x3 | Synergie |
| **Cx1/Acide fusidique** | 8/2 | 64/8 | 0,125-1 | 0,5/0,125 | ↓ x4 | ↓ x4 | Synergie |
| **Cx1/Fosfomycine** | 8/8 | 64/32 | 0,18-1 | 0,5/1 | ↓ x4 | ↓ x3 | Synergie |

**3.2. Tableau VI : Synergie vis-à-vis d'une souche de SARM sans résistance associée (ou SaR1) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/0,5 | 64/4 | 0,5-1 | 2/0,125 | ↓ x2 | ↓ x2 | Additivité |
| **CxI/Imipénème** | 8/0,25 | 64/4 | 0,625-1 | 0,5/0,06 | ↓ x4 | ↓ x2 | Additivité |
| **Cx1/Erythromycine** | 8/0,5 | 64/4 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/0,25 | 64/4 | 1-4 | nd | nd | nd | Indifférence |
| **Cx1/Gentamicine** | 8/0,25 | 64/2 | 0,49-1 | 2/0,06 | ↓ x2 | ↓ x2 | Additivité |
| **Cx1/Streptomycine** | 8/4 | 64/32 | 0,75-1 | 4/1 | ↓ x1 | ↓ x2 | Additivité |
| **Cx1/Linézolide** | 8/2 | 64/8 | 1-4 | nd | nd | nd | Indifférence |
| **Cx1/Tigécycline** | 8/0,25 | 64/2 | 0,18-1 | 0,5/0,03 | ↓ x4 | ↓ x3 | Synergie |
| **Cx1/Acide fusidique** | 8/0,5 | 64/8 | 0,245-1 | 1/0,06 | ↓ x3 | ↓ x3 | Synergie |
| **Cx1/Fosfomycine** | 8/2 | 64/32 | 0,31-1 | 0,5/0,5 | ↓ x4 | ↓ x2 | Synergie |

**3.3. Tableau VII : Synergie vis-à-vis d'une souche de SARM présentant une résistance aux aminosides et aux fluoroquinolones (SaR3) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/0,5 | 64/4 | 0,375-1 | 2/0,06 | ↓ x2 | ↓ x3 | Synergie |
| **Cxl/Imipénème** | 8/0,25 | 64/4 | 0,56-1 | 2/0,06 | ↓ x2 | ↓ x2 | Additivité |
| **Cx1/Erythromycine** | 8/0,5 | 64/4 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/8 | 64/32 | 1-4 | nd | nd | nd | Indifférence |
| **Cx1/Gentamicine** | 8/0,125 | 64/2 | 0,74-1 | 4/0,03 | ↓ x1 | ↓ x2 | Additivité |
| **Cx1/Streptomycine** | 8/4 | 64/32 | 0,625-1 | 1/2 | ↓ x3 | ↓ x1 | Additivité |
| **Cx1/Linézolide** | 8/2 | 64/8 | 1-4 | nd | nd | nd | Indifférence |
| **Cx1/Tigécycline** | 8/0,25 | 64/2 | 0,245-1 | 1/0,03 | ↓ x3 | ↓ x3 | Synergie |
| **Cx1/Acide fusidique** | 8/0,25 | 64/8 | 0,365-1 | 1/0,06 | ↓ x3 | ↓ x2 | Synergie |
| **Cx1/Fosfomycine** | 8/16 | 64/32 | 0,18-1 | 0,5/2 | ↓ x4 | ↓ x3 | Synergie |

**3.4. Tableau VIII : Synergie vis-à-vis d'une souche de SARM présentant une résistance aux aminosides, aux fluoroquinolones, aux macrolides-lincosamides-synergistines et à l'ofloxacine (SaR4) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Pénicilline G** | 8/4 | 64/32 | 0,375-1 | 2/0,5 | ↓ x2 | ↓ x3 | Synergie |
| **Cxl/Imipénème** | 8/2 | 64/32 | 0,375-1 | 1/0,5<br>2/0,25 | ↓ x3<br>↓ x2 | ↓ x2<br>↓ x3 | Synergie |
| **Cx1/Erythromycine** | 8/>32 | 64/32 | 1-4 | nd | nd | nd | Indifférence |
| **Cx1/Vancomycine** | 8/0,25 | 64/4 | 1-4 | nd | nd | nd | Indifférence |
| **Cx1/Lévofloxacine** | 8/>32 | 64/32 | 1-4 | nd | nd | nd | Indifférence |
| **Cx1/Gentamicine** | 8/0,25 | 64/2 | 0,30-1 | 0,5/0,06 | ↓ x4 | ↓ x2 | Synergie |
| **Cx1/Streptomycine** | 8/4 | 64/32 | 0,625-1 | 4/0,5 | ↓ x1 | ↓ x3 | Additivité |
| **Cx1/Linézolide** | 8/2 | 64/8 | 0,56-1 | 0,5/1 | ↓ x4 | ↓ x1 | Additivité |
| **Cx1/Tigécycline** | 8/0,25 | 64/2 | 0,18-1 | 0,5/0,03 | ↓ x4 | ↓ x3 | Synergie |
| Cx1/Acide fusidique | 8/4 | 64/8 | 0,08-1 | 0,5/0,06 | ↓ x4 | ↓ x6 | Synergie |
| Cx1/Fosfomycine | 8/128 | 64/32 | 1-4 | nd | nd | nd | Indifférence |

**4. Synergie de l'association du Cx1 avec des antibiotiques vis-à-vis de souches de *Pseudomonas aeruginosa* résistantes ou non aux antibiotiques**

**4.1. Tableau IX : Synergie vis-à-vis de la souche *Pseudomonas aeruginosa* ATCC 27853 (phénotype sauvage) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/8 | 256/32 | 0,375-1 | 4/1 | ↓ x3 | ↓ x3 | Synergie |
| **Cx1/Pipéracilline-Tazobactam** | 32/16 | 256/128 | 0,31-1 | 2/4 | ↓ x4 | ↓ x2 | Synergie |
| **Cx1/Ceftazidime** | 32/4 | 256/64 | 0,375-1 | 4/1 | ↓ x2 | ↓ x2 | Synergie |
| **Cxl/Imipénème** | 32/2 | 256/16 | 0,5-4 | 8/0,5 | x↓2 | ↓ x2 | Additivité |
| **Cx1/Rifampicine** | 32/64 | 256/256 | 0,12-1 | 2/4 | ↓x4 | ↓ x4 | Synergie |
| **Cx1/Colimycine** | 32/4 | 256/64 | 0,5-1 | nd | nd | ↓nd | Additivité |
| **Cx1/Fosfomycine** | 32/16 | 256/256 | 0,375-1 | 4/4<br>2/8 | ↓ x3<br>↓ x4 | ↓ x2<br>↓ x1 | Synergie |
| **Cx1/Tobramycine** | 32/0,5 | 256/4 | 0,18-1 | 2/0,06 | ↓ x4 | ↓ x3 | Synergie |
| **Cx1/Amikacine** | 32/0,5 | 256/4 | 1-4 | nd | nd | nd | Indifférence |
| **Cx1/Ciprofloxacine** | 32/0,5 | 256/4 | 0,185-1 | 4/0,125 | ↓ x3 | ↓ x2 | Synergie |

**4.2. Tableau X : Synergie vis-à-vis d'une souche de *Pseudomonas aeruginosa* présentant une résistance aux β-lactamines, à l'association triméthoprime-sulfaméthoxazole et à la fosfomycine (ou PaR2) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/512 | 256/512 | 1-4 | nd | nd | nd | Indifférence |
| **Cx1/Pipéracilline-Tazobactam** | 32/512 | 256/512 | 0,25-1 | 4/64 | ↓ x3 | ↓ x3 | Synergie |
| **Cx1/Ceftazidime** | 32/8 | 256/64 | 1-4 | nd | nd | nd | Indifférence |
| **Cxl/Imipénème** | 32/2 | 256/16 | 0,5-4 | 8/1 | ↓ x2 | x1 | Additivité |
| **Cx1/Rifampicine** | 32/64 | 256/256 | 0,185-1 | 4/4<br>8/2 | ↓ x3<br>↓ x2 | ↓ x4<br>↓ x5 | Synergie |
| **Cx1/Colimycine** | 32/8 | 256/64 | 1-4 | nd | nd | nd | Indifférence |
| **Cx1/Fosfomycine** | 32/64 | 256/256 | 0,5-4 | 16/32 | ↓ x1 | ↓ x1 | Additivité |
| **Cx1/Tobramycine** | 32/1 | 256/4 | 0,31-1 | 2/0,25 | ↓ x4 | ↓ x2 | Synergie |
| **Cx1/Amikacine** | 32/1 | 256/4 | 1-4 | nd | nd | nd | Indifférence |
| **Cx1/Ciprofloxacine** | 32/1 | 256/4 | 0,5-4 | nd | nd | nd | Additivité |

**4.3. Tableau XI : Synergie vis-à-vis d'une souche de *Pseudomonas aeruginosa* présentant une résistance aux β-lactamines (y compris les carbapénèmes), aux aminosides, à l'association triméthoprime-sulfaméthoxazole et à la ciprofloxacine (ou PaR3) (n = 4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/512 | 256/512 | 1-4 | nd | nd | nd | Indifférence |
| **Cx1/Pipéracilline-Tazobactam** | 32/512 | 256/512 | 1-4 | nd | nd | nd | Indifférence |
| **Cx1/Ceftazidime** | 32/16 | 256/64 | 0,31-1 | 2/4 | ↓ x4 | ↓ x2 | Synergie |
| **Cx1/Imipénème** | 32/32 | 256/256 | 0,5-1 | 8/8 | ↓ x2 | ↓ x2 | Additivité |
| **Cx1/Rifampicine** | 32/32 | 256/256 | 0,09-1 | 2/2 | ↓ x4 | ↓ x4 | Synergie |
| **Cx1/Colimycine** | 32/8 | 256/64 | 0,155-1 | 4/0,5 | ↓ x3 | ↓ x4 | Synergie |
| **Cx1/Fosfomycine** | 32/> 64 | 256/256 | 0,31-1 | 2/64 | ↓ x4 | ↓ x2 | Synergie |
| **Cx1/Tobramycine** | 32/64 | 256/256 | 0,185-1 | 2/8 | ↓ x4 | ↓ x3 | Synergie |
| **Cx1/Amikacine** | 32/1 | 256/4 | 1-4 | nd | nd | nd | Indifférence |
| **Cx1/Ciprofloxacine** | 32/0,5 | 256/4 | 0,5/4 | 8/0,125 | ↓ x2 | ↓ x2 | Additivité |

**4.4. Tableau XII : Synergie vis-à-vis d'une souche muqueuse de *Pseudomonas aeruginosa* présentant une résistance à la rifampicine et à l'association triméthoprime-sulfaméthoxazole (ou PaR5) (n=4)**

| Associations | CMI (mg/L) | Gammes testées (mg/L) | FIC index | Concentrations optimales (mg/L) | Différence CMI Cx1 | Différence CMI ATB | Conclusion |
|---|---|---|---|---|---|---|---|
| **Cx1/Ticarcilline-Acide clavulanique** | 32/32 | 256/512 | 1-4 | nd | nd | nd | Indifférence |
| **Cx1/Pipéracilline-Tazobactam** | 32/32 | 256/512 | 0,31-1 | 2/8 | ↓ x4 | ↓ x2 | Synergie |
| **Cx1/Ceftazidime** | 32/8 | 256/64 | 1-4 | nd | nd | nd | Indifférence |
| **Cxl/Imipénème** | 32/2 | 256/16 | 0,25-1 | 4/0,25 | ↓ x3 | ↓ x3 | Synergie |
| **Cx1/Rifampicine** | 32/16 | 256/256 | 0,185-1 | 2/2 | ↓ x4 | ↓ x3 | Synergie |
| **Cx1/Colimycine** | 32/16 | 256/64 | 0,187-1 | 2/2 | ↓ x4 | ↓ x3 | Synergie |
| **Cx1/Fosfomycine** | 32/16 | 256/256 | 0,375-1 | 8/2<br>4/4 | ↓ x2<br>↓ x3 | ↓ x3<br>↓ x2 | Synergie |
| **Cx1/Tobramycine** | 32/1 | 256/4 | 0,25-1 | 4/0,125 | ↓ x3 | ↓ x3 | Synergie |
| **Cx1/Amikacine** | 32/0,5 | 256/4 | 1-4 | nd | nd | nd | Indifférence |
| Cx1/Ciprofloxacine | 32/1 | 256/4 | 0,31-1 | 2/0,25 | ↓ x4 | ↓ x2 | Synergie |

**5. Identification et antibiogramme**

**5.1 EcR1 : *Escherichia coli* productrice de pénicillinase sans résistance associée**

L'identification bactérienne et d'antibiogramme est effectuée par le système VITEK2 (bioMérieux) et par la technique de diffusion sur disques.

Tableau A1

| Antibiotiques | Diamètre | Dmin Dmax | Résultats | CMI mg/L | Résultats |
|---|---|---|---|---|---|
| Amoxicilline | 6 | 14-21 | Résistant | ≥ 32 | Résistant |
| Amox + ac.clavulanique | 20 | 14-21 | Intermédiaire | 4 | Sensible |
| Ticarcilline | 6 | 18-22 | Résistant | ≥ 128 | Résistant |
| Pipéracilline | 17 | 12-20 | Intermédiaire | ≤ 8 | Sensible |
| Piper + tazobactam | 25 | 14-21 | Sensible | ≤ 4 | Sensible |
| C1G | 17 | 12-18 | Intermédiaire | 4 | Sensible |
| Céfoxitine | 24 | 15-22 | Sensible | ≤ 4 | Sensible |
| Céfotaxime | 30 | 15-21 | Sensible | ≤ 1 | Sensible |
| Ceftazidime | | | | ≤ 1 | Sensible |
| Imipénème | | | | ≤ 1 | Sensible |
| Aztréonam | 28 | 17-23 | Sensible | | |
| Tobramycine | 19 | 14-16 | Sensible | ≤ 1 | Sensible |
| Gentamicine | 20 | 14-16 | Sensible | ≤ 1 | Sensible |
| Amikacine | 19 | 15-17 | Sensible | ≤ 2 | Sensible |
| Netilmicine | 24 | 17-19 | Sensible | ≤ 1 | Sensible |
| Minocycline | 20 | 17-19 | Sensible | | |
| Colistine | 15 | 15 | Sensible | | |
| Trimétoprime - Sulfamét. | 21 | 10-16 | Sensible | ≤ 20 | Sensible |
| Acide nalidixique | | | | ≤ 2 | Sensible |
| Norfloxacine | | | | ≤ 0,5 | Sensible |
| Ofloxacine | | | | ≤ 0,25 | Sensible |
| Péfloxacine | 26 | 16-22 | Sensible | | |
| Ciprofloxacine | 27 | 19-22 | Sensible | ≤ 0,25 | Sensible |
| Rifampicine | 16 | 14-19 | Intermédiaire | | |
| Fosfomycine | 24 | 14 | Sensible | | |
| Nitrofurantoïne | | | | ≤ 16 | Sensible |
| Céfépime | 27 | 15-21 | Sensible | | |

Tableau A2

| Antibiotiques | CMI mg/L (diamètre en mm) | Cc CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Amoxicilline | ≥ 32 | 4-8 | **Résistant** | 8 | **Résistant** | 8-32 | **Résistant** |
| Amox + ac.clavulanique | 4 | 4-8 | Sensible | 8 | Sensible | 8-32 | Sensible |
| Ticarcilline | ≥ 128 | 8-16 | **Résistant** | 8-16 | **Résistant** | 16-128 | **Résistant** |
| Pipéracilline | ≤ 8 | 8-16 | Sensible | 8-16 | Sensible | 16-128 | Sensible |
| Piper + tazobactam | ≤ 4 | 8-16 | Sensible | 8-16 | Sensible | 16-128 | Sensible |
| Céfalotine | 4 | 8-32 | Sensible | 16 | Sensible | 8-32 | Sensible |
| Céfoxitine | ≤ 4 | 8-32 | Sensible | NA | | 8-32 | Sensible |
| Céfotaxime | ≤ 1 | 1-2 | Sensible | 1-2 | Sensible | 1-4 | Sensible |
| Ceftazidime | ≤ 1 | 1-4 | Sensible | 1-4 | Sensible | 4-16 | Sensible |
| Céfépime | 27 | 24 | Sensible | 21-24 | Sensible | 14-18 | Sensible |
| Imipénème | ≤ 0.5 | 0.5-1 | Sensible | 2-8 | Sensible | 4-16 | Sensible |
| Aztréonam | 28 | 21-27 | Sensible | 24-27 | Sensible | 17-21 | Sensible |
| Tobramycine | ≤ 1 | 2-4 | Sensible | 2-4 | Sensible | 4-16 | Sensible |
| Gentamicine | ≤ 1 | 2-4 | Sensible | 2-4 | Sensible | 4-16 | Sensible |
| Amikacine | ≤ 2 | 8-16 | Sensible | 8-16 | Sensible | 16-64 | Sensible |
| Netilmicine | ≤ 1 | 2-4 | Sensible | 2-4 | Sensible | 8-32 | Sensible |
| Minocycline | 20 | 17-19 | Sensible | - | - | 12-16 | Sensible |
| Colistine | 15 | 15 | Sensible | 17 | Résistant | nd | nd |
| Trimétoprime - Sulfamét. | ≤ 20 | 2-4 | Sensible | 2-4 | Sensible | 2-4 | Sensible |
| Acide nalidixique | ≤ 2 | 8-16 | Sensible | NA | - | 16-32 | Sensible |
| Norfloxacine | ≤ 0,5 | 0.5-1 | Sensible | 0.5-1 | Sensible | 4-16 | Sensible |
| Ofloxacine | ≤ 0,25 | 0.5-1 | Sensible | 0.5-1 | Sensible | 2-8 | Sensible |
| Ciprofloxacine | ≤ 0,25 | 0.5-1 | Sensible | - | - | 1-4 | Sensible |
| Rifampicine | 16 | 14-19 | **Intermédiaire** | - | - | nd | nd |
| Fosfomycine | 24 | 14 | Sensible | - | - | 12-16 | Sensible |
| Nitrofurantoïne | ≤ 16 | 64 | Sensible | 64 | Sensible | 32-128 | Sensible |

*Cc : Concentration critique

Conclusion expert juillet 2006 : Pénicillinase acquise

Les fluctuations relatives au type de résistance associée pour certaines souches entre les tableaux A1 et A2 sont dues à l'évolution des recommandations françaises (CA-SFM), européennes (EUCAST) et américaines (CLSI) en matière de catégorisation des souches bactériennes.

5.2 **EcR2 : *Escherichia coli* hyperproductrice de céphalosporinase présentant une résistance associée aux aminosides, aux quinolones et à l'association triméthoprime-sulfaméthoxazole**

L'identification bactérienne et d'antibiogramme est effectuée par le système VITEK1 (bioMérieux) et par la technique de diffusion sur disques

Tableau B1

| Antibiotiques | Diamètre | Dmin Dmax | Résultats | CMI mg/L |
|---|---|---|---|---|
| Amoxicilline | | | Résistant | ≥ 32 |
| Amox + ac.clavulanique | | | Résistant | ≥ 32 |
| Ticarcilline | | | Intermédiaire | 32 |
| C1G | | | Résistant | ≥ 64 |
| Céfoxitine | 11 | 15-22 | Résistant | |
| Céfotaxime | 24 | 15-21 | Intermédiaire | |
| Ceftazidime | 20 | 15-21 | Intermédiaire | |
| Imipénème | | | Sensible | ≤ 4 |
| Tobramycine | 10 | 16-18 | Résistant | |
| Gentamicine | 12 | 16-18 | Résistant | |
| Netilmicine | 21 | 19-21 | Sensible | ≤ 1 |
| Trimétoprime - Sulfamét. | | | Intermédiaire | 160 |
| Acide nalidixique | | | Résistant | ≥ 32 |
| Péfloxacine | | | Résistant | ≥ 8 |
| Nitrofurantoïne | | | Sensible | ≤ 25 |

Tableau B2

| Antibiotiques | CMI mg/L (diamètre en mm) | Cc*CA -SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Amoxicilline | ≥ 32 | 4-8 | **Résistant** | 8 | **Résistant** | 8-32 | **Résistant** |
| Amox + ac.clavulanique | ≥ 32 | 4-8 | **Résistant** | 8 | **Résistant** | 8-32 | **Résistant** |
| Ticarcilline | 32 | 8-16 | **Résistant** | 8-16 | **Résistant** | 16-128 | **Résistant** |
| Céfalotine | ≥ 64 | 8-32 | **Résistant** | 16 | **Résistant** | 8-32 | **Résistant** |
| Céfoxitine | 11 | 15-22 | **Résistant** | 19 | **Résistant** | 14-18 | **Résistant** |
| **Céfotaxime | 24 | 23-26 | **Intermédiaire** | 18-21 | **Résistant** | 22-26 | **Intermédiaire** |
| **Ceftazidime | 20 | 23-26 | **Résistant** | 19-22 | **Intermédiaire** | 17-21 | **Intermédiaire** |
| Imipénème | ≤ 0.5 | 0.5-1 | Sensible | 2-8 | Sensible | 4-16 | Sensible |
| Tobramycine | 10 | 16-18 | **Résistant** | 13-16 | **Résistant** | 12-15 | **Résistant** |
| **Gentamicine | 12 | 16-18 | **Résistant** | 14-17 | **Résistant** | 12-15 | **Résistant** |
| Netilmicine | ≤ 1 | 2-4 | Sensible | 2-4 | Sensible | 8-32 | Sensible |
| Trimétoprime - Sulfamét. | 160 | 2-4 | **Résistant** | 2-4 | **Résistant** | 8-16 | **Résistant** |
| Acide nalidixique | ≥ 32 | 8-16 | **Résistant** | - | - | 16-32 | **Résistant** |
| Péfloxacine | ≥ 8 | 1-4 | **Résistant** | - | - | - | - |
| Nitrofurantoïne | ≤ 25 | 64 | Sensible | 64 | Sensible | 32-128 | Sensible |

*Cc : Concentration critique

** Attention : la charge du disque est différente entre CA-SFM et EUCAST ; l'interprétation de l'EUCAST n'est donc pas applicable dans le présent cas. De plus, la technique de réalisation du test de diffusion en disque est différente dans les deux référentiels.

Les fluctuations relatives au type de résistance associée pour certaines souches entre les tableaux B1 et B2 sont dues à l'évolution des recommandations françaises (CA-SFM), européennes (EUCAST) et américaines (CLSI) en matière de catégorisation des souches bactériennes.

5.3 **EcR3 : *Escherichia coli* productrice de BLSE présentant une résistance associée aux aminosides, à la rifampicine et à l'association triméthoprime-sulfaméthoxazole**

L'identification bactérienne et d'antibiogramme est effectuée par le système VITEK2 (bioMérieux) et par la technique de diffusion sur disques

Tableau C1

| Antibiotiques | Diamètre | Dmin Dmax | Résultats |
|---|---|---|---|
| Amoxicilline | 6 | 14-21 | Résistant |
| Amox + ac.clavulanique | 16 | 14-21 | Intermédiaire |
| Ticarcilline | 6 | 18-22 | Résistant |
| Pipéracilline | 13 | 12-20 | Intermédiaire |
| Piper + tazobactam | | | Intermédiaire |
| C1G | 6 | 12-18 | Résistant |
| Céfoxitine | | | Intermédiaire |
| Céfotaxime | 19 | 15-21 | Intermédiaire |
| Imipénème | 27 | 17-22 | Sensible |
| Tobramycine | 6 | 14-16 | Résistant |
| Gentamicine | 12 | 14-16 | Résistant |
| Amikacine | 17 | 15-17 | Sensible |
| Netilmicine | 10 | 17-19 | Résistant |
| Minocycline | 21 | 17-19 | Sensible |
| Colistine | 16 | 15 | Sensible |
| Trimétoprime - Sulfamét. | 6 | 10-16 | Résistant |
| Péfloxacine | 23 | 16-22 | Sensible |
| Ciprofloxacine | 25 | 19-22 | Sensible |
| Rifampicine | 15 | 14-19 | Intermédiaire |
| Fosfomycine | 23 | 14 | Sensible |
| Céfépime | 24 | 15-21 | Intermédiaire |

Tableau C2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Amoxicilline | 6 | 16-19 | **Résistant** | 14 | **Résistant** | 13-17 | **Résistant** |
| Amox + ac.clavulanique | 16 | 16-21 | **Intermédiaire** | 17 | **Résistant** | 13-18 | **Intermédiaire** |
| Ticarcilline | 6 | 22-24 | **Résistant** | 22-23 | **Résistant** | 14-20 | **Résistant** |
| Pipéracilline | 13 | 16-20 | **Résistant** | 15-18 | **Résistant** | 17-21 | **Résistant** |
| **Piper + tazobactam | 18 | 17-21 | **Intermédiaire** | 15-18 | Sensible | 17-21 | **Intermédiaire** |
| Céfalotine | 6 | 12-18 | **Résistant** | - | - | 14-18 | **Résistant** |
| Céfoxitine | 6 | 15-22 | **Résistant** | 19 | **Résistant** | 14-18 | **Résistant** |
| **Céfotaxime | 19 | 23-26 | **Résistant** | 18-21 | **Intermédiaire** | 22-26 | **Résistant** |
| Céfépime | 24 | 24 | **Sensible** | 21-24 | Sensible | 14-18 | Sensible |
| Imipénème | 27 | 17-24 | Sensible | 15-21 | Sensible | 13-16 | Sensible |
| Tobramycine | 6 | 16-18 | **Résistant** | 13-16 | **Résistant** | 12-15 | **Résistant** |
| **Gentamicine | 12 | 16-18 | **Résistant** | 14-17 | **Résistant** | 12-15 | **Intermédiaire** |
| Amikacine | 17 | 15-17 | Sensible | 13-16 | Sensible | 14-17 | Sensible |
| **Netilmicine | 10 | 19-21 | **Résistant** | 12-15 | **Résistant** | 12-15 | **Résistant** |
| Minocycline | 21 | 17-19 | Sensible | - | - | 12-16 | Sensible |
| Colistine | 16 | 15 | Sensible | - | - | - | - |
| Trimétoprime - Sulfamét. | 6 | 13-16 | **Résistant** | 13-16 | **Résistant** | 10-16 | **Résistant** |
| Péfloxacine | 23 | 16-22 | Sensible | - | - | - | - |
| Ciprofloxacine | 25 | 22-25 | Sensible | - | - | 15-21 | Sensible |
| Rifampicine | 15 | 14-19 | **Intermédiaire** | - | - | - | - |
| Fosfomycine | 23 | 14 | Sensible | - | - | 12-16 | Sensible |

*Dc : Diamètre critique

**Attention : la charge du disque est différente entre CA-SFM et EUCAST ; l'interprétation de l'EUCAST n'est pas applicable dans le cas présent. De plus, la technique de réalisation du test de diffusion en disque est différente dans les deux référentiels.

Les fluctuations relatives au type de résistance associée pour certaines souches entre les tableaux C1 et C2 sont dues à l'évolution des recommandations françaises (CA-SFM), européennes (EUCAST) et américaines (CLSI) en matière de catégorisation des souches bactériennes.

5.4 **SaR1 : *Staphylococcus aureus* Résistant à la Méticilline sans résistance associée**

L'identification bactérienne et d'antibiogramme est effectuée par le système VITEK2 (bioMérieux)

Tableau D1

| Antibiotiques | Diamètre | Dmin Dmax | Résultats | CMI mg/L | Résultats |
|---|---|---|---|---|---|
| Pénicilline G | 28 | 9-29 | Résistant | ≥ 0,5 | Résistant |
| Oxacilline | | | | ≥ 8 | Résistant |
| Kanamycine | 20 | 15-17 | Sensible | ≤ 4 | Sensible |
| Tobramycine | 22 | 14-16 | Sensible | ≤ 1 | Sensible |
| Gentamicine | 23 | 14-16 | Sensible | ≤ 0,5 | Sensible |
| Chloramphénicol | 25 | 19-23 | Sensible | | |
| Minocycline | 27 | 17-19 | Sensible | | |
| Erythromycine | 25 | 17-22 | Sensible | ≤ 0,25 | Sensible |
| Lincomycine | 24 | 17-21 | Sensible | ≤ 1 | Sensible |
| Pristinamycine | 25 | 19-22 | Sensible | ≤ 0,5 | Sensible |
| Quinupristine-dalfopristine | | | | ≤ 0,25 | Sensible |
| Trimétoprime - Sulfamét. | 26 | 10-16 | Sensible | ≤ 10 | Sensible |
| Ofloxacine | 24 | 16-22 | Sensible | 1 | Sensible |
| Acide fusidique | 28 | 15-22 | Sensible | ≤ 0,5 | Sensible |
| Vancomycine | | | Sensible | ≤ 1 | Sensible |
| Teicoplanine | | | Sensible | ≤ 0,5 | Sensible |
| Rifampicine | 30 | 14-29 | Sensible | ≤ 0,5 | Sensible |
| Fosfomycine | 40 | 14 | Sensible | ≤ 8 | Sensible |
| Linézolide | 29 | 24-28 | Sensible | 2 | Sensible |
| Minocycline | | | | ≤ 0,5 | Sensible |
| Nitrofurantoïne | | | | ≤ 16 | Sensible |

Tableau D2

| Antibiotiques | CMI mg/L | Cc*CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Pénicilline G | ≥ 0,5 | 0,12 | **Résistant** | 0,125 | **Résistant** | 0,12-0,25 | **Résistant** |
| Oxacilline | ≥ 8 | 2 | **Résistant** | 2 | **Résistant** | 2-4 | **Résistant** |
| Kanamycine | ≤ 4 | 8-16 | Sensible | 8-16 | Sensible | 16-64 | Sensible |
| Tobramycine | ≤ 1 | 1 | Sensible | 1 | Sensible | 4-16 | Sensible |
| Gentamicine | ≤ 0,5 | 1 | Sensible | 1 | Sensible | 4-16 | Sensible |
| Erythromycine | ≤ 0,25 | 1-2 | Sensible | 1-2 | Sensible | 0,5-8 | Sensible |
| Lincomycine | ≤ 1 | 2-8 | Sensible | - | - | - | - |
| Pristinamycine | ≤ 0,5 | 1-2 | Sensible | - | - | - | - |
| Quinupristine-dalfopristine | ≤ 0,25 | 1-2 | Sensible | 1-2 | Sensible | 1-4 | Sensible |
| Trimétoprime-Sulfamét. | ≤ 10 | 2-4 | Sensible | 2-4 | Sensible | 2-4 | Sensible |
| Ofloxacine | 1 | 1 | Sensible | 1 | Sensible | 1-4 | Sensible |
| Acide fusidique | ≤ 0,5 | 1 | Sensible | 1 | Sensible | - | - |
| Vancomycine | ≤ 1 | 2 | Sensible | 2 | Sensible | 4-32 | Sensible |
| Teicoplanine | ≤ 0,5 | 4 | Sensible | 2 | Sensible | 8-32 | Sensible |
| Rifampicine | ≤ 0,5 | 0,06-0,5 | Sensible | 0,064-0,5 | Sensible | 1-4 | Sensible |
| Fosfomycine | ≤ 8 | 32 | Sensible | 32 | Sensible | - | - |
| Linézolide | 2 | 4 | Sensible | 4 | Sensible | 4-8 | Sensible |
| Minocycline | ≤ 0,5 | 0,5-1 | Sensible | 0,5-1 | Sensible | 4-16 | Sensible |
| Nitrofurantoïne | ≤ 16 | 64 | Sensible | 64 | Sensible | 32-128 | Sensible |

* Cc : Concentration critique

Février 2006 : Recherche du gène mecA par technique PCR : POSITIVE Conclusion expert juillet 2006 : phénotype tout à fait typique : modification des PLP

Les fluctuations relatives au type de résistance associée pour certaines souches entre les tableaux D1 et D2 sont dues à l'évolution des recommandations françaises (CA-SFM), européennes (EUCAST) et américaines (CLSI) en matière de catégorisation des souches bactériennes.

### 5.5 **SaR3 : *Staphylococcus aureus* Résistant à la Méticilline présentant une résistance associée aux aminosides et aux fluoroquinolones**

L'identification bactérienne et d'antibiogramme est effectuée par le système VITEK2 (bioMérieux) et par la technique de diffusion sur disques

Tableau E1

| Antibiotiques | Diamètre | Dmin Dmax | Résultats |
|---|---|---|---|
| Pénicilline G | 26 | 9-29 | Résistant |
| Oxacilline | | | |
| Kanamycine | 6 | 15-17 | Résistant |
| Tobramycine | 6 | 14-16 | Résistant |
| Gentamicine | 20 | 14-16 | Sensible |
| Chloramphénicol | 6 | 19-23 | Résistant |
| Minocycline | 26 | 17-19 | Sensible |
| Erythromycine | 25 | 17-22 | Sensible |
| Lincomycine | 23 | 17-21 | Sensible |
| Pristinamycine | 23 | 19-22 | Sensible |
| Trimétoprime - Sulfamét. | 27 | 10-16 | Sensible |
| Ofloxacine | 6 | 16-22 | Résistant |
| Acide fusidique | 29 | 15-22 | Sensible |
| Vancomycine | | | Sensible |
| Teicoplanine | | | Sensible |
| Rifampicine | 31 | 14-29 | Sensible |
| Fosfomycine | 26 | 14 | Sensible |
| Linézolide | 26 | 24-28 | Sensible |

Tableau E2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Pénicilline G | 26 | | **Résistant** | 26 | **Résistant** | 28-29 | **Résistant** |
| Céfoxitine | 17 | 25-37 | **Résistant** | 22 | **Résistant** | 21-22 | **Résistant** |
| **Kanamycine | 6 | 15-17 | **Résistant** | 16-18 | **Résistant** | 13-18 | **Résistant** |
| Tobramycine | 6 | 20 | **Résistant** | 18 | **Résistant** | 12-15 | **Résistant** |
| **Gentamicine | 20 | 20 | Sensible | 18 | Sensible | 12-15 | Sensible |
| Erythromycine | 25 | 19-22 | Sensible | 18-21 | Sensible | 13-23 | Sensible |
| Lincomycine | 23 | 17-21 | Sensible | - | - | - | - |
| Pristinamycine | 23 | 19-22 | Sensible | - | - | - | - |
| Trimétoprime - Sulfamét. | 27 | 13-16 | Sensible | 14-17 | Sensible | 10-16 | Sensible |
| Ofloxacine | 6 | 22 | **Résistant** | 20 | **Résistant** | 14-18 | **Résistant** |
| Acide fusidique | 29 | 24 | Sensible | 24 | Sensible | | - |
| Vancomycine | 28 | 17 | Sensible | - | - | - | - |
| Teicoplanine | 27 | 17 | Sensible | - | - | 10-14 | Sensible |
| Rifampicine | 31 | 24-29 | Sensible | 23-26 | Sensible | 16-20 | Sensible |
| Fosfomycine | 26 | 14 | Sensible | - | - | - | - |
| **Linézolide | 26 | 24 | Sensible | 19 | Sensible | 20-21 | Sensible |
| Minocycline | 26 | 21-23 | Sensible | 20-23 | Sensible | 14-19 | Sensible |

* Dc : Diamètre critique

** Attention : la charge du disque est différente entre CA-SFM et EUCAST ; l'interpétation d'EUCAST n'est dons pas applicable dans le cas présent. De plus, la technique de réalisation du test de diffusion en disque est différente dans les deux référentiels.

Les fluctuations relatives au type de résistance associée pour certaines souches entre les tableaux E1 et E2 sont dues à l'évolution des recommandations françaises (CA-SFM), européennes (EUCAST) et américaines (CLSI) en matière de catégorisation des souches bactériennes.

5.6 **SaR4 : *Staphylococcus aureus* Résistant à la Méticilline présentant une résistance associée aux aminosides, aux fluoroquinolones, aux macrolides-lincosamines-synergistines et à l'ofloxacine**

L'identification bactérienne et d'antibiogramme est effectuée par le système VITEK2 (bioMérieux) et par la technique de diffusion sur disques

Tableau F1

| Antibiotiques | Diamètre | Dmin Dmax | Résultats | CMI mg/L |
|---|---|---|---|---|
| Pénicilline G | | | Résistant | |
| Oxacilline | | | Résistant | ≥ 8 |
| Kanamycine | 7 | 15-17 | Résistant | |
| Tobramycine | 6 | 20-20 | Résistant | |
| Gentamicine | 21 | 20-20 | Sensible | |
| Chloramphénicol | | | Sensible | 8 |
| Tétracycline | | | Sensible | ≤ 1 |
| Minocycline | | | Sensible | <4 |
| Erythromycine | | | Résistant | ≥ 8 |
| Lincomycine | | | Résistant | >16 |
| Pristinamycine | | | Sensible | <2 |
| Trimétoprime - Sulfamét. | | | Sensible | <10 |
| Ofloxacine | | | Résistant | ≥ 8 |
| Nitrofurantoïne | | | Sensible | ≤ 25 |
| Acide fusidique | | | Sensible | ≤ 1 |
| Vancomycine | | | Sensible | 1 |
| Teicoplanine | | | Sensible | ≤ 4 |
| Rifampicine | | | Sensible | ≤ 1 |
| Fosfomycine | | | Résistant | ≥ 64 |

Tableau F2

| **Antibiotiques** | **CMI mg/L (diamètre en mm)** | **Cc*CA-SFM 2011 mg/L** | **Interprétation CA-SFM** | **Cc EUCAST 2011** | **Interprétation EUCAST** | **Cc CLSI 2011** | **Interprétation CLSI** |
|---|---|---|---|---|---|---|---|
| Pénicilline G | | | **Résistant** | | **Résistant** | 0,12-0,25 | **Résistant** |
| Oxacilline | ≥ 8 | 2 | **Résistant** | 2 | **Résistant** | 2-4 | **Résistant** |
| **Kanamycine | 7 | 15-17 | **Résistant** | 16-18 | **Résistant** | 13-18 | **Résistant** |
| Tobramycine | 6 | 20 | **Résistant** | 18 | **Résistant** | 12-15 | **Résistant** |
| **Gentamicine | 21 | 20 | Sensible | 18 | Sensible | 12-15 | Sensible |
| Tétracycline | ≤ 1 | 1-2 | Sensible | 1-2 | Sensible | 4-16 | Sensible |
| Minocycline | ≤ 0,5 | 0,5-1 | Sensible | 0,5-1 | Sensible | 4-16 | Sensible |
| Erythromycine | ≥ 8 | 1-2 | **Résistant** | 1-2 | **Résistant** | 0,5-8 | **Résistant** |
| Lincomycine | ≥ 16 | 2-8 | **Résistant** | - | - | - | - |
| Pristinamycine | ≤ 2 | 1-2 | Sensible | - | - | - | - |
| Trimétoprim - Sulfamét. | ≤ 10 | 2-4 | Sensible | 2-4 | Sensible | 2-4 | Sensible |
| Ofloxacine | ≥ 8 | 1 | Résistant | 1 | Résistant | 1-4 | Résistant |
| Nitrofurantoïne | ≤ 25 | 64 | Sensible | 64 | Sensible | 32-128 | Sensible |
| Acide fusidique | ≤ 1 | 1 | Sensible | 1 | Sensible | | |
| Vancomycine | 1 | 2 | Sensible | 2 | Sensible | 4-32 | Sensible |
| Teicoplanine | ≤ 4 | 4 | Sensible | 2 | Sensible | 8-32 | Sensible |
| Rifampicine | ≤ 1 | 0,06-0,5 | Sensible | 0,064-0,5 | Sensible | 1-4 | Sensible |
| Fosfomycine | ≥ 64 | 32 | Résistant | 32 | Résistant | | |

*Cc : Concentration critique

** Attention : la charge du disque est différente entre CA-SFM et EUCAST ; l'interprétation de l'EUCAST n'est donc pas applicable dans le cas présent. De plus, la technique de réalisation du test de diffusion en disque est différente dans les deux référentiels.

Les fluctuations relatives au type de résistance associée pour certaines souches entre les tableaux F1 et F2 sont dues à l'évolution des recommandations françaises (CA-SFM), européennes (EUCAST) et américaines (CLSI) en matière de catégorisation des souches bactériennes.

5.7 **PaR2 : *Pseudomonas aeruginosa* présentant une résistance associée aux β-lactamines, à l'association triméthoprime-sulfaméthoxazole et à la fosfomycine**

L'identification bactérienne et d'antibiogramme est effectuée par le système VITEK2 (bioMérieux) et par la technique de diffusion sur disques

Tableau G1

| Antibiotiques | Diamètre | Dmin-Dmax | Résultats |
|---|---|---|---|
| Ticar + ac. clavulanique | 10 | 18-22 | Résistants |
| Ticarcilline | 10 | 18-22 | Résistant |
| Pipéracilline | 22 | 12-18 | Sensible |
| Piper + tazobactam | 22 | 14-19 | Sensible |
| Ceftazidime | 23 | 15-21 | Sensible |
| Aztréonam | 16 | 17-23 | Résistant |
| Imipénème | 27 | 17-22 | Sensible |
| Tobramycine | 24 | 14-16 | Sensible |
| Gentamicine | 21 | 14-16 | Sensible |
| Amikacine | 22 | 15-17 | Sensible |
| Nétilmicine | 19 | 17-19 | Sensible |
| Minocycline | 6 | 17-19 | Résistant |
| Colistine | 20 | 15-15 | Sensible |
| Trimétoprime - Sulfamét. | 6 | 10-16 | Résistant |
| Péfloxacine | 8 | 16-22 | Résistant |
| Rifampicine | 25 | 19-22 | Sensible |
| Fosfomycine | 13 | 14-19 | Résistant |
| Ciprofloxacine | 18 | 14-14 | Sensible |
| Céfépime | 19 | 15-21 | Intermédiaire |

Tableau G2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Ticar + ac. clavulanique | 10 | 22 | **Résistant** | 17 | **Résistant** | 14-15 | **Résistant** |
| Ticarcilline | 10 | 22 | **Résistant** | 17 | **Résistant** | 14-15 | **Résistant** |
| **Pipéracilline | 22 | 18 | Sensible | 19 | Sensible | 17-18 | Sensible |
| **Piper + tazobactam | 22 | 19 | Sensible | 19 | Sensible | 17-18 | Sensible |
| **Ceftazidime | 23 | 19 | Sensible | 16 | Sensible | 14-18 | Sensible |
| Céfépime | 19 | 19 | Sensible | 18 | Sensible | 14-18 | Sensible |
| Aztréonam | 16 | 19-27 | **Résistant** | 16-50 | **Résistant** | 15-22 | **Intermédiaire** |
| Imipénème | 27 | 17-22 | Sensible | 17-20 | Sensible | 13-16 | Sensible |
| Tobramycine | 24 | 16 | Sensible | 16 | Sensible | 12-15 | Sensible |
| **Gentamicine | 21 | 16 | Sensible | 15 | Sensible | 12-15 | Sensible |
| Amikacine | 22 | 15-17 | Sensible | 15-18 | Sensible | 14-17 | Sensible |
| **Nétilmicine | 19 | 19 | Sensible | 12 | Sensible | 12-15 | Sensible |
| Minocycline | 6 | - | **Résistant** | - | - | - | - |
| Colistine | 20 | - | Sensible | - | - | 10-11 | Sensible |
| Trimétoprime-Sulfamét. | 6 | - | **Résistant** | 16 | **Résistant** | - | - |
| Ciprofloxacine | 25 | 22-25 | Sensible | 22-25 | Sensible | 15-21 | Sensible |
| Rifampicine | 25 | 14-19 | Sensible | - | - | - | - |
| Fosfomycine | 13 | 14 | **Résistant** | - | - | - | - |

*Dc : Diamètre critique

** Attention : la charge du disque est différente entre CA-SFM et EUCAST; l'interprétation de l'EUCAST n'est donc pas applicable dans le cas présent. De plus, la technique de réalisation du test de diffusion en disque est différente dans les deux référentiels.

Les fluctuations relatives au type de résistance associée pour certaines souches entre les tableaux G1 et G2 sont dues à l'évolution des recommandations françaises (CA-SFM), européennes (EUCAST) et américaines (CLSI) en matière de catégorisation des souches bactériennes.

5.8 **PaR3 : *Pseudomonas aeruginosa* présentant une résistance associée aux β-lactamines**, **aux aminosides (y compris les carbapénèmes**), **à l'association triméthoprime**-**sulfaméthoxazole et à la ciprofloxacine**

L'identification bactérienne et d'antibiogramme est effectuée par le système VITEK2 (bioMérieux)

Tableau H1

| Antibiotiques | CMI | Résultats |
|---|---|---|
| Ticar + ac. clavulanique | ≥ 128 | Résistant |
| Ticarcilline | ≥ 128 | Résistant |
| Pipéracilline | ≥ 128 | Résistant |
| Piper + tazobactam | ≥ 128 | Résistant |
| Ceftazidime | 4 | Sensible |
| Aztréonam | 16 | Sensible |
| Imipénème | ≥ 16 | Résistant |
| Méropénème | ≥ 16 | Résistant |
| Tobramycine | ≥ 16 | Résistant |
| Gentamicine | ≥ 16 | Résistant |
| Amikacine | 4 | Sensible |
| Minocycline | 4 | Résistant |
| Colistine | ≤ 0,5 | Sensible |
| Trimétoprime - Sulfamét. | ≥ 320 | Résistant |
| Péfloxacine | 4 | Intermédiaire |
| Ciprofloxacine | 1 | Sensible |
| Céfépime | 16 | Intermédiaire |

Tableau H2

| Antibiotiques | CMI mg/L | Cc*CA-SFM 2011 mg/L | Interprétation CA-SFM | Cc EUCAST 2011 | Interprétation EUCAST | Cc CLSI 2011 | Interprétation CLSI |
|---|---|---|---|---|---|---|---|
| Ticar + ac. clavulanique | ≥ 128 | 16 | **Résistant** | 16 | **Résistant** | 64-128 | **Résistant** |
| Ticarcilline | ≥ 128 | 16 | **Résistant** | 16 | **Résistant** | 64-128 | **Résistant** |
| Pipéracilline | ≥ 128 | 16 | **Résistant** | 16 | **Résistant** | 64-128 | **Résistant** |
| Piper + tazobactam | ≥ 128 | 16 | **Résistant** | 16 | **Résistant** | 64-128 | **Résistant** |
| Ceftazidime | 4 | 8 | Sensible | 8 | Sensible | 8-32 | Sensible |
| Céfépime | 16 | 8 | **Résistant** | 8 | **Résistant** | 8-32 | **Intermédiaire** |
| Aztréonam | 16 | 1-16 | **Résistant** | 1-16 | **Résistant** | 8-32 | **Intermédiaire** |
| Imipénème | ≥ 16 | 4-8 | **Résistant** | 4-8 | **Résistant** | 4-16 | **Résistant** |
| Méropénème | ≥ 16 | 2-8 | **Résistant** | 2-8 | **Résistant** | 4-16 | **Résistant** |
| Tobramycine | ≥ 16 | 4 | **Résistant** | 4 | **Résistant** | 4-16 | **Résistant** |
| Gentamicine | ≥ 16 | 4 | **Résistant** | 4 | **Résistant** | 4-16 | **Résistant** |
| Amikacine | 4 | 8-16 | Sensible | 8-16 | Sensible | 16-64 | Sensible |
| Minocycline | 4 | - | **Résistant** | - | - | - | - |
| Colistine | ≤ 0,5 | 2-4 | Sensible | 4 | Sensible | 2-8 | Sensible |
| Trimétoprime - Sulfamét. | ≥ 320 | - | **Résistant** | 4 | **Résistant** | - | - |
| Ciprofloxacine | 1 | 0,5-1 | **Intermédiaire** | 0,5-1 | **Intermédiaire** | 1-4 | Sensible |

*Cc : concentration critique

Les fluctuations relatives au type de résistance associée pour certaines souches entre les tableaux H1 et H2 sont dues à l'évolution des recommandations françaises (CA-SFM), européennes (EUCAST) et américaines (CLSI) en matière de catégorisation des souches bactériennes.

5.9 **PaR5 : *Pseudomonas aeruginosa* présentant une résistance associée à la rifampicine et à l'association triméthoprime-sulfaméthoxazole**

L'identification bactérienne et d'antibiogramme est effectuée par le système VITEK2 (bioMérieux) et par la technique de diffusion sur disques

Tableau I1

| Antibiotiques | Diamètre | Dmin-Dmax | Résultats |
|---|---|---|---|
| Ticar + ac. clavulanique | 40 | 18-22 | Sensible |
| Ticarcilline | 32 | 18-22 | Sensible |
| Pipéracilline | 33 | 12-18 | Sensible |
| Piper + tazobactam | 40 | 14-19 | Sensible |
| Ceftazidime | 32 | 15-21 | Sensible |
| Aztréonam | 33 | 17-23 | Sensible |
| Imipénème | 25 | 17-22 | Sensible |
| Méropénème | 34 | 15-20 | Sensible |
| Tobramycine | 28 | 14-16 | Sensible |
| Gentamicine | 25 | 14-16 | Sensible |
| Amikacine | 27 | 15-17 | Sensible |
| Nétilmicine | 28 | 17-19 | Sensible |
| Minocycline | 11 | 17-19 | Résistant |
| Colistine | 23 | 15 | Sensible |
| Trimétoprime - Sulfamét. | 6 | 10-16 | Résistant |
| Norofloxacine | 17 | 22-25 | Résistant |
| Ofloxacine | 11 | 22-25 | Résistant |
| Ciprofloxacine | 30 | 19-22 | Sensible |
| Rifampicine | 13 | 14-19 | Résistant |
| Fosfomycine | 30 | 14 | Sensible |
| Céfépime | 32 | 15-21 | Sensible |

Tableau I2

| Antibiotiques | diamètre en mm | Dc* CA-SFM 2011 | Interprétation CA-SFM | Dc EUCAST 2011 | Interprétation EUCAST | Dc CLSI 2011 | Interprétation CLSI |
|---|---|---|---|---|---|---|---|
| Ticar + ac. clavulanique | 40 | 22 | Sensible | 17 | Sensible | 14-15 | Sensible |
| Ticarcilline | 32 | 22 | Sensible | 17 | Sensible | 14-15 | Sensible |
| **Pipéracilline | 33 | 18 | Sensible | 19 | Sensible | 17-18 | Sensible |
| **Piper + tazobactam | 40 | 19 | Sensible | 19 | Sensible | 17-18 | Sensible |
| **Ceftazidime | 32 | 19 | Sensible | 16 | Sensible | 14-18 | Sensible |
| Céfépime | 32 | 19 | Sensible | 18 | Sensible | 14-18 | Sensible |
| Aztréonam | 33 | 19-27 | Sensible | 16-50 | **Intermédiaire** | 15-22 | Sensible |
| Imipénème | 25 | 17-22 | Sensible | 17-20 | Sensible | 13-16 | Sensible |
| Méropénème | 34 | 15-22 | Sensible | 18-24 | Sensible | 13-16 | Sensible |
| Tobramycine | 28 | 16 | Sensible | 16 | Sensible | 12-15 | Sensible |
| **Gentamicine | 25 | 16 | Sensible | 15 | Sensible | 12-15 | Sensible |
| Amikacine | 27 | 15-17 | Sensible | 15-18 | Sensible | 14-17 | Sensible |
| **Nétilmicine | 28 | 19 | Sensible | 12 | Sensible | 12-15 | Sensible |
| Minocycline | 11 | - | **Résistant** | - | - | - | - |
| Colistine | 23 | - | Sensible | - | - | 10-11 | Sensible |
| Trimétoprime - Sulfamét. | 6 | - | **Résistant** | 16 | **Résistant** | - | - |
| Ciprofloxacine | 30 | 22-25 | Sensible | 22-25 | Sensible | 15-21 | Sensible |
| Rifampicine | 13 | 14-19 | **Résistant** | - | - | - | - |
| Fosfomycine | 30 | 14 | Sensible | - | - | - | - |

*Dc : diamètre critique

**Attention : la charge du disque est différente entre CA-SFM et EUCAST; l'interprétation de l'EUCAST n'est donc pas applicable dans le cas présent. De plus la technique de réalisation du test de diffusion en disque est différente dans les deux référentiels.

Les fluctuations relatives au type de résistance associée pour certaines souches entre les tableaux I1 et I2 sont dues à l'évolution des recommandations françaises (CA-SFM), européennes (EUCAST) et américaines (CLSI) en matière de catégorisation des souches bactériennes.

**6. Synergie de l'association du Cx1 avec des antiseptiques vis-à-vis des souches bactériennes de phénotype sauvage**

**6.1. Synergie vis-à-vis de la souche d'*E. coli* ATCC 25922 (phénotype sauvage)**

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 4/8 | 64/32 | >1 | nd | nd | nd | Indifférence |
| **Cx1/Chlorhexidine** | 4/<1 | 64/4 | >1 | nd | nd | nd | Indifférence |

6.2 Synergie vis-à-vis de la souche *S. aureus* ATCC 29213 (phénotype sauvage)

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 8/<1 | 64/4 | >1 | nd | nd | nd | Indifférence |
| **Cx1/Chlorhexidine** | 8/<1 | 64/4 | >1 | nd | nd | nd | Indifférence |

6.3. Synergie vis-à-vis de la souche *P. aeruginosa* ATCC 27853 (phénotype sauvage)

| Associations | CMI initiales (mg/L) | Gammes testées (mg/L) | FIC index | CMI optimales (mg/L) | Différence CMI Cx1 | Différence CMI ATS | Conclusion |
|---|---|---|---|---|---|---|---|
| **Cx1/Hexamidine** | 32/32 | 64/256 | 3 | nd | nd | nd | Indifférence |
| **Cx1/Chlorhexidine** | 32/4 | 64/32 | 1.5 | nd | nd | nd | Indifférence |

**Revendications**

1. Produit comprenant un calixarène représenté par la formule I suivante :

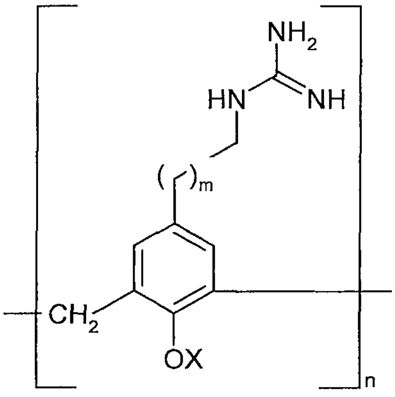

Formule I

,

dans lequel :

(i) n = un entier de 4 à 16,
(ii) m= un entier de 1 à 10,
(iii) X est choisi parmi :

- un hydrogène,
- un groupement alkyl, le nombre de carbones étant de 1 à 20, notamment de 1 à 10,
- un halogène choisi parmi Cl, Br, I, ou
- un groupement amphiphile choisi parmi un groupe anionique, tels que les carboxylates $-RCO_2^-$, les sulfates $-RSO_4^-$, les sulfonates $-RSO_3^-$, un groupe cationique, tel que $RNH_3^+$, dans lesquels R est un groupement alkyl, le nombre de carbones étant de 1 à 20, notamment de 1 à 10,

pour son utilisation dans le traitement de pathologies impliquant au moins une souche bactérienne présentant une résistance à au moins un antibiotique déterminé, sur des patients sous traitement simultané ou séquentiel par un antibiotique donné vis-à-vis duquel ladite souche bactérienne présente éventuellement une résistance, ou
pour son utilisation dans le traitement de pathologies impliquant une souche bactérienne de phénotype sauvage ne présentant de résistance acquise à aucun antibiotique connu, sur des patients sous traitement simultané ou séquentiel par un « antibiotique donné » vis-à-vis duquel ladite souche bactérienne ne présente pas de résistance.

2. Produit pour utilisation selon la revendication 1, dans le traitement de pathologies impliquant une souche bactérienne présentant une résistance acquise à au moins un antibiotique déterminé, sur des patients sous traitement simultané ou séquentiel par ledit antibiotique.

3. Produit pour utilisation selon la revendication 1, dans le traitement de pathologies impliquant au moins une souche bactérienne présentant une résistance acquise à au moins un antibiotique déterminé, sur des patients sous traitement simultané ou séquentiel par un antibiotique donné vis-à-vis duquel ladite souche bactérienne présente une résistance acquise.

4. Produit pour utilisation selon la revendication 1, dans le traitement de pathologies impliquant au moins une souche bactérienne présentant une résistance acquise à au moins un antibiotique déterminé, sur des patients sous traitement simultané ou séquentiel par un antibiotique donné vis-à-vis duquel ladite souche bactérienne est sensible.

5. Produit pour utilisation selon la revendication 1, dans le traitement de pathologies impliquant au moins une souche bactérienne présentant une résistance acquise à au moins un antibiotique déterminé, sur des patients sous traitement simultané ou séquentiel par un antibiotique donné vis-à-vis duquel ladite souche bactérienne présente une résistance naturelle.

6. Produit pour utilisation selon la revendication 1, dans le traitement de pathologies impliquant une souche bactérienne

présentant une résistance naturelle à au moins un antibiotique déterminé, sur des patients sous traitement simultané ou séquentiel par ledit antibiotique.

**7.** Produit pour utilisation selon la revendication 1, dans le traitement de pathologies impliquant au moins une souche bactérienne présentant une résistance naturelle à au moins un antibiotique déterminé, sur des patients sous traitement simultané ou séquentiel par un antibiotique donné vis-à-vis duquel ladite souche bactérienne présente une résistance naturelle.

**8.** Produit pour utilisation selon la revendication 1, dans le traitement de pathologies impliquant au moins une souche bactérienne présentant une résistance naturelle à au moins un antibiotique déterminé, sur des patients sous traitement simultané ou séquentiel par un antibiotique donné vis-à-vis duquel ladite souche bactérienne est sensible.

**9.** Produit pour utilisation selon la revendication 1, dans le traitement de pathologies impliquant au moins une souche bactérienne présentant une résistance naturelle à au moins un antibiotique déterminé, sur des patients sous traitement simultané ou séquentiel par un antibiotique donné vis-à-vis duquel ladite souche bactérienne présente une résistance acquise.

**10.** Produit pour utilisation selon l'une des revendications 1 à 9, comprenant un calixarène représenté par la formule I, dans laquelle :

- n=4,
- m=1,
- X est un hydrogène,

ledit calixarène correspondant au calixarène représenté par la formule II suivante :

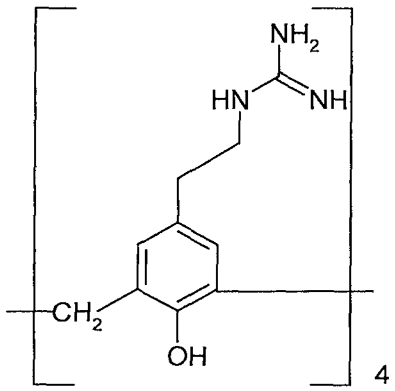

Formule II

**11.** Produit pour utilisation selon l'une des revendications 1 à 10, dans le traitement des pathologies appartenant au groupe constitué des infections nosocomiales et/ou communautaires, telles que les infections abdominales, les infections digestives, les infections urinaires, les infections respiratoires, les infections neuro-méningées, les infections de la sphère oro-pharyngée, les infections génitales, les endocardites, les infections de la peau et des tissus mous, les infections ostéo-articulaires, les infections oculaires, les septicémies ou bactériémies.

**12.** Produit pour utilisation selon l'une des revendications 1 à 11, dans le traitement des pathologies impliquant une souche bactérienne résistante appartenant à une espèce choisie parmi *Escherichia coli, Pseudomonas aeruginosa,* et *Staphylococcus aureus.*

**13.** Produit pour utilisation selon la revendication 12, dans le traitement des pathologies impliquant souche bactérienne résistante choisie parmi :

- une souche de *Staphylococcus aureus* de phénotype sauvage,
- une souche de *Staphylococcus aureus* Résistant à la Méticilline (SARM) sans résistance associée,
- une souche de SARM présentant une résistance aux aminosides et aux fluoroquinolones,
- une souche de SARM présentant une résistance aux aminosides, aux fluoroquinolones, aux macrolides-lincosamides-synergystines et à l'ofloxacine,

- une souche d'*Escherichia coli* de phénotype sauvage,
- une souche d'*Escherichia coli* productrice de BLSE (β-Lactamase à Spectre Etendu) présentant une résistance associée aux aminosides, à la rifampicine et à l'association triméthoprime-sulfaméthoxazole,
- une souche d'*Escherichia coli* productrice de pénicillinase sans résistance associée,
- une souche d'*Escherichia coli* hyperproductrice de céphalosporinase présentant une résistance associée aux aminosides, aux quinolones et à l'association triméthoprime-sulfaméthoxazole,
- une souche de *Pseudomonas aeruginosa* de phénotype sauvage,
- une souche de *Pseudomonas aeruginosa* présentant une résistance aux β-lactamines, à l'association triméthoprime-sulfaméthoxazole et à la fosfomycine,
- une souche de *Pseudomonas aeruginosa* présentant une résistance aux β-lactamines (y compris les carbapénèmes), aux aminosides, à l'association triméthoprime-sulfaméthoxazole et à la ciprofloxacine,
- une souche muqueuse de *Pseudomonas aeruginosa* présentant une résistance à la rifampicine et à l'association triméthoprime-sulfaméthoxazole.

**14.** Produit pour utilisation selon l'une des revendications 1 à 13, dans lequel ledit antibiotique donné est choisi dans le groupe constitué des β- lactamines, des aminosides, des fluoroquinolones, de la fosfomycine, de la colimycine, de la rifampicine, de la tigécycline, ou de l'acide fusidique.

**15.** Produit pour utilisation selon la revendication 14, dans lequel ledit antibiotique donné est choisi parmi l'imipénème, la pipéracilline-tazobactam, la pénicilline G, le céfotaxime, la ceftazidime, la tobramycine, la gentamicine, la ciprofloxacine, la rifampicine, la fosfomycine, la colimycine, la tigécycline, la ticarcilline-acide clavulanique, la streptomycine ou l'acide fusidique.

**Patentansprüche**

**1.** Produkt, das ein Calixaren umfasst, welches durch die folgende Formel I dargestellt wird

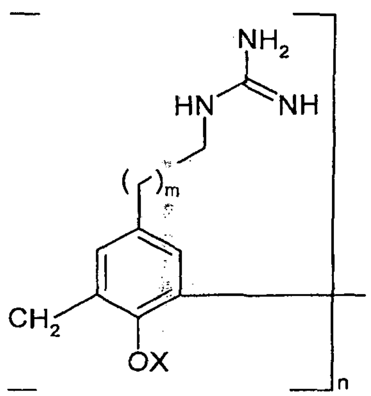

Formel I

Wobei

(i) n = eine ganze Zahl von 4 bis 16,
(ii) m = eine ganze Zahl von 1 bis 10,
(iii) X ausgewählt ist aus:

- Wasserstoff,
- einer Alkylgruppe, wobei die Anzahl der Kohlenstoffe 1 bis 20 beträgt, insbesondere 1-10,
- einem Halogen, ausgewählt aus Cl, Br, I,
oder
- einer amphiphilen Gruppe, ausgewählt aus einer anionischen Gruppe, wie etwa Carboxylaten - $RCO_2^-$, Sulfaten -$RSO_4^-$, Sulfonaten -$RSO_3^-$, einer kationischen Gruppe, wie etwa $RNH_3^+$, wobei R eine Alkylgruppe ist, wobei die Anzahl von Kohlenstoffen 1 bis 20 beträgt, insbesondere 1 bis 10,

zur Verwendung bei der Behandlung von Erkrankungen, die mit mindestens einem Bakterienstamm mit einer Resistenz gegen mindestens ein bestimmtes Antibiotikum einhergehen, bei Patienten unter gleichzeitiger oder sequenzieller Therapie mit einem gegebenen Antibiotikum, gegenüber welchem der Bakterienstamm gegebenenfalls eine Resistenz aufweist, oder

zur Verwendung bei der Behandlung von Erkrankungen, die mit mindestens einem Bakterienstamm mit wildtypischem Phänotyp einhergehen, der keine erworbene Resistenz gegen kein bekanntes Antibiotikum aufweist, bei Patienten unter gleichzeitiger oder sequenzieller Therapie mit einem "gegebenen Antibiotikum", gegenüber welchem der Bakterienstamm keine Resistenz aufweist.

**2.** Produkt zur Verwendung nach Anspruch 1 bei der Behandlung von Erkrankungen, die mit einem Bakterienstamm einhergehen, der eine erworbene Resistenz gegen mindestens ein bestimmtes Antibiotikum aufweist, bei Patienten unter gleichzeitiger oder sequentieller Behandlung mit dem Antibiotikum.

**3.** Produkt zur Verwendung nach Anspruch 1 bei der Behandlung von Erkrankungen, die mit mindestens einem Bakterienstamm einhergehen, der eine erworbene Resistenz gegen mindestens ein bestimmtes Antibiotikum aufweist, bei Patienten unter gleichzeitiger oder sequentieller Behandlung mit einem gegebenen Antibiotikum, gegenüber welchem der Bakterienstamm eine erworbene Resistenz aufweist.

**4.** Produkt zur Verwendung nach Anspruch 1 bei der Behandlung von Erkrankungen, die mit mindestens einem Bakterienstamm einhergehen, der eine erworbene Resistenz gegen mindestens ein bestimmtes Antibiotikum aufweist, bei Patienten unter gleichzeitiger oder sequentieller Behandlung mit einem gegebenen Antibiotikum, gegenüber welchem der Bakterienstamm sensibel ist.

**5.** Produkt zur Verwendung nach Anspruch 1, bei der Behandlung von Erkrankungen, die mit mindestens einem Bakterienstamm einhergehen, der eine erworbene Resistenz gegen mindestens ein bestimmtes Antibiotikum aufweist bei Patienten unter gleichzeitiger oder sequentieller Behandlung mit einem gegebenen Antibiotikum, gegenüber welchem der Bakterienstamm eine natürliche Resistenz aufweist.

**6.** Produkt zur Verwendung nach Anspruch 1, bei der Behandlung von Erkrankungen, die mit mindestens einem Bakterienstamm einhergehen, der eine natürliche Resistenz gegen mindestens ein bestimmtes Antibiotikum aufweist, bei Patienten unter gleichzeitiger oder sequentieller Behandlung mit dem Antibiotikum.

**7.** Produkt zur Verwendung nach Anspruch 1, bei der Behandlung von Erkrankungen, die mit mindestens einem Bakterienstamm einhergehen, der eine natürliche Resistenz gegen mindestens ein bestimmtes Antibiotikum aufweist, bei Patienten unter gleichzeitiger oder sequentieller Behandlung mit einem gegebenen Antibiotikum, gegenüber welchem der Bakterienstamm eine natürliche Resistenz aufweist.

**8.** Produkt zur Verwendung nach Anspruch 1, bei der Behandlung von Erkrankungen, die mit mindestens einem Bakterienstamm einhergehen, der eine natürliche Resistenz gegen mindestens ein bestimmtes Antibiotikum aufweist, bei Patienten unter gleichzeitiger oder sequentieller Behandlung mit einem gegebenen Antibiotikum, gegenüber welchem der Bakterienstamm sensibel ist.

**9.** Produkt zur Verwendung nach Anspruch 1, bei der Behandlung von Erkrankungen, die mit mindestens einem Bakterienstamm einhergehen, der eine natürliche Resistenz gegen mindestens ein bestimmtes Antibiotikum aufweist, bei Patienten unter gleichzeitiger oder sequentieller Behandlung mit einem gegebenen Antibiotikum, gegenüber welchem der Bakterienstamm eine erworbene Resistenz aufweist.

**10.** Produkt zur Verwendung nach einem der Ansprüche 1 bis 9, das ein Calixaren umfasst welches durch die Formel I dargestellt wird, wobei:

- n = 4,
- m=1,
- X ein Wasserstoff ist,

wobei das Calixaren dem durch die folgende Formel II dargestellten Calixaren entspricht:

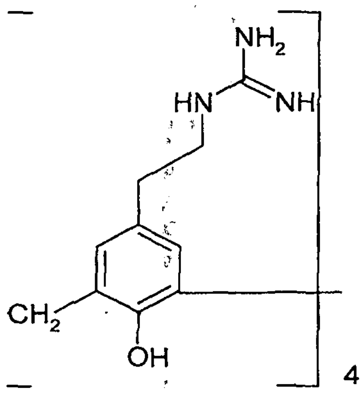

Formel II

11. Produkt zur Verwendung nach einem der Ansprüche 1 bis 10, bei der Behandlung von Erkrankungen der Gruppe bestehend aus nosokomialen und/oder Gemeinschaftsinfektionen, wie etwa abdominalen Infektionen, gastrointestinalen Infektionen, Harnwegsinfektionen, Atemwegsinfektionen, Neuro-Meningitis Infektionen, Infektionen des Mund-Rachen-Bereichs, Genitalinfektionen, Endokarditis, Infektionen der Haut und der Weichgewebe, Knochen- und Gelenksinfektionen, Augeninfektionen, Sepsen oder Bakteriämien.

12. Produkt zur Verwendung nach einem der Ansprüche 1 bis 11 bei der Behandlung von Erkrankungen, die mit einem resistenten Bakterienstamm einhergehen, der einer Spezies ausgewählt unter *Escherichia coli, Pseudomonas aeruginosa* und *Staphylococcus aureus* angehört.

13. Produkt zur Verwendung nach Anspruch 12 bei der Behandlung von Erkrankungen, die mit einem resistenten Bakterienstamm einhergehen, der ausgewählt ist aus:

- einem *Staphylococcus aureus* Stamm mit wildtypischem Phänotyp,
- einem *Staphylococcus aureus* Stamm resistent gegen Methicillin (MRSA) ohne assoziierte Resistenz,
- einem MRSA Stamm, der eine Resistenz gegen Aminoglykoside und Fluorchinolone aufweist,
- einem MRSA Stamm, der eine Resistenz gegen Aminoglykoside, Fluorchinolone, Makrolide-Lincosamide-Synergystine und gegen Ofloxacin aufweist,
- einem *Escherichia coli* Stamm mit wildtypischem Phänotyp,
- einem ESBL produzierenden (β-Lactamase mit breitem Wirkungsspektrum) *Escherichia coli* Stamm mit einer Resistenz gegen Aminoglykoside, Rifampicin und die Verbindung Trimethoprim-Sulfamethoxazol,
- einem Penicillinase produzierenden *Escherichia coli* Stamm ohne assoziierte Resistenz,
- einem Cephalosporinase hochproduzierenden *Escherichia coli* Stamm mit einer Resistenz gegen Aminoglykoside, Chinolone und die Verbindung Trimethoprim-Sulfamethoxazol,
- einem *Pseudomonas aeruginosa* Stamm mit wildtypischem Phänotyp,
- einem *Pseudomonas aeruginosa* Stamm mit einer Resistenz gegen β-Lactam-Antibiotika, die Verbindung Trimethoprim-Sulfamethoxazol und Fosfomycin,
- einem *Pseudomonas aeruginosa* Stamm mit einer Resistenz gegen β-Lactam-Antibiotika (einschließlich Carbapeneme), Aminoglykoside, die Verbindung Trimethoprim-Sulfamethoxazol und Ciprofloxacin,
- einem *Pseudomonas aeruginosa* Schleimhaut-Stamm mit einer Resistenz gegen Rifampicin und die Verbindung Trimethoprim-Sulfamethoxazol.

14. Produkt zur Verwendung nach einem der Ansprüche 1 bis 13, wobei das gegebene Antibiotikum ausgewählt ist aus der Gruppe bestehend aus β-Lactam-Antibiotika, Aminoglykosiden, Fluorchinolonen, Fosfomycin, Colimycin, Rifampicin, Tigecyclin oder Fusidinsäure.

15. Produkt zur Verwendung nach Anspruch 14, wobei das gegebene Antibiotikum ausgewählt ist aus Imipenem, Piperacillin-Tazobactam, Penicillin G, Cefotaxim, Ceftazidim, Tobramycin, Gentamycin, Ciprofloxacin, Rifampicin, Fosfomycin, Colimycin, Tigecyclin, Ticarcillin-Clavulansäure, Streptomycin oder Fusidinsäure.

**Claims**

1. Product comprising a calixarene represented by Formula I below:

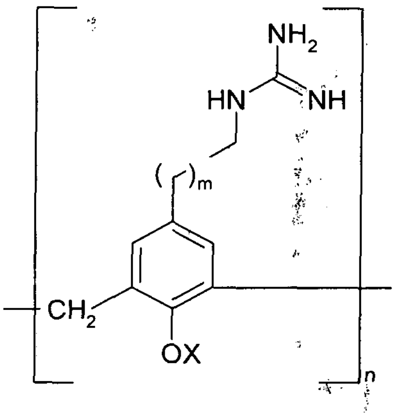

Formula I

,

in which:

(i) n = an integer from 4 to 16,
(ii) m = an integer from 1 to 10,
(iii) X is chosen from:

- a hydrogen,
- an alkyl group, the number of carbons being from 1 to 20, in particular from 1 to 10,
- a halogen chosen from Cl, Br, I, or
- an amphiphilic group chosen from an anionic group, such as the carboxylates $-RCO_2^-$, the sulphates $-RSO_4^-$, the sulphonates $-RSO_3^-$, a cationic group, such as $RNH_3^+$, in which R is an alkyl group, the number of carbons being from 1 to 20, in particular from 1 to 10,

for its use in the treatment of pathologies involving at least one bacterial strain having a resistance to at least one defined antibiotic, in patients undergoing simultaneous or sequential treatment with a given antibiotic to which said bacterial strain optionally has a resistance, or for its use in the treatment of pathologies involving a wild-type bacterial strain having no acquired resistance to any known antibiotic, in patients undergoing simultaneous or sequential treatment with a "given antibiotic" to which said bacterial strain has no resistance.

**2.** Product for use according to claim 1, in the treatment of pathologies involving a bacterial strain having an acquired resistance to at least one defined antibiotic, in patients undergoing simultaneous or sequential treatment by said antibiotic.

**3.** Product for use according to claim 1, in the treatment of pathologies involving at least one bacterial strain having an acquired resistance to at least one defined antibiotic, in patients undergoing simultaneous or sequential treatment with a given antibiotic to which said bacterial strain has an acquired resistance.

**4.** Product for use according to claim 1, in the treatment of pathologies involving at least one bacterial strain having an acquired resistance to at least one defined antibiotic, in patients undergoing simultaneous or sequential treatment with a given antibiotic to which said bacterial strain is susceptible.

**5.** Product for use according to claim 1, in the treatment of pathologies involving at least one bacterial strain having an acquired resistance to at least one defined antibiotic, in patients undergoing simultaneous or sequential treatment with a given antibiotic to which said bacterial strain has a natural resistance.

**6.** Product for use according to claim 1, in the treatment of pathologies involving a bacterial strain having a natural resistance to at least one defined antibiotic, in patients undergoing simultaneous or sequential treatment with said antibiotic.

**7.** Product for use according to claim 1, in the treatment of pathologies involving at least one bacterial strain having a natural resistance to at least one defined antibiotic, in patients undergoing simultaneous or sequential treatment with a given antibiotic to which said bacterial strain has a natural resistance.

**8.** Product for use according to claim 1, in the treatment of pathologies involving at least one bacterial strain having a natural resistance to at least one defined antibiotic, in patients undergoing simultaneous or sequential treatment with a given antibiotic to which said bacterial strain is susceptible.

**9.** Product for use according to claim 1, in the treatment of pathologies involving at least one bacterial strain having a natural resistance to at least one defined antibiotic, in patients undergoing simultaneous or sequential treatment with a given antibiotic to which said bacterial strain has an acquired resistance.

**10.** Product for use according to one of claims 1 to 9, comprising a calixarene represented by Formula I, in which:

- n = 4,
- m = 1,
- X is a hydrogen,

said calixarene corresponding with the calixarene represented by Formula II below:

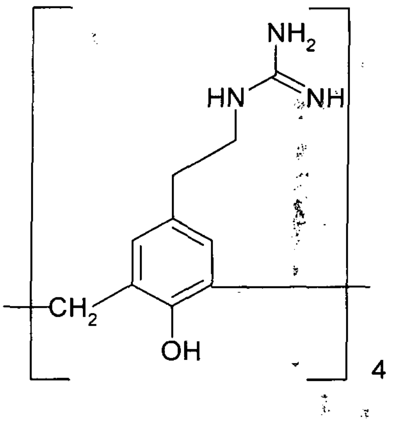

Formula II

**11.** Product for use according to one of claims 1 to 10, in the treatment of the pathologies belonging to the group constituted by nosocomial and/or community-acquired infections, such as abdominal infections, digestive infections, urinary infections, respiratory infections, neuro-meningeal infections, infections of the oro-pharyngeal sphere, genital infections, endocarditis, infections of the skin and soft tissues, osteoarticular infections, ocular infections, septicaemia or bacteriaemia.

**12.** Product for use according to one of claims 1 to 11, in the treatment of the pathologies involving a resistant bacterial strain belonging to a species chosen from *Escherichia coli*, *Pseudomonas aeruginosa*, and *Staphylococcus aureus.*

**13.** Product for use according to claim 12, in the treatment of the pathologies involving a resistant bacterial strain chosen from:

- a wild-type strain of *Staphylococcus aureus,*
- a strain of meticillin-resistant *Staphylococcus aureus* (MRSA) without associated resistance,
- a strain of MRSA having a resistance to the aminoglycosides and fluoroquinolones,
- a strain of MRSA having a resistance to the aminoglycosides, fluoroquinolones, macrolides-lincosamides-synergystins and ofloxacin,
- a wild-type strain of *Escherichia coli,*
- an ESBL (extended spectrum β-Lactamase)-producing strain of *Escherichia coli,* having an associated resistance to the aminoglycosides, rifampicin and the trimethoprime-sulphamethoxazole combination,
- a penicillinase-producing strain of *Escherichia coli* without associated resistance,
- a cephalosporinase-hyperproducing strain of *Escherichia coli* having an associated resistance to the aminoglycosides, quinolones and the trimethoprime-sulphamethoxazole combination,
- a wild-type strain of *Pseudomonas aeruginosa*,
- a strain of *Pseudomonas aeruginosa* having a resistance to the β-lactams, the trimethoprime-sulphamethoxazole combination and fosfomycin,
- a strain of *Pseudomonas aeruginosa* having a resistance to the β-lactams (including the carbapenems),

aminoglycosides, the trimethoprime-sulphamethoxazole combination and ciprofloxacin,
- a mucoid strain of *Pseudomonas aeruginosa* having a resistance to rifampicin and the trimethoprime-sulphamethoxazole combination.

**14.** Product for use according to one of claims 1 to 13, in which said given antibiotic is chosen from the group constituted by the β-lactams, aminoglycosides, fluoroquinolones, fosfomycin, colimycin, rifampicin, tigecycline, or fusidic acid.

**15.** Product for use according to claim 14, in which said given antibiotic is chosen from imipenem, piperacillin-tazobactam, penicillin G, cefotaxime, ceftazidime, tobramycin, gentamicin, ciprofloxacin, rifampicin, fosfomycin, colimycin, tigecycline, ticarcillin-clavulanic acid, streptomycin or fusidic acid.

Figure 1

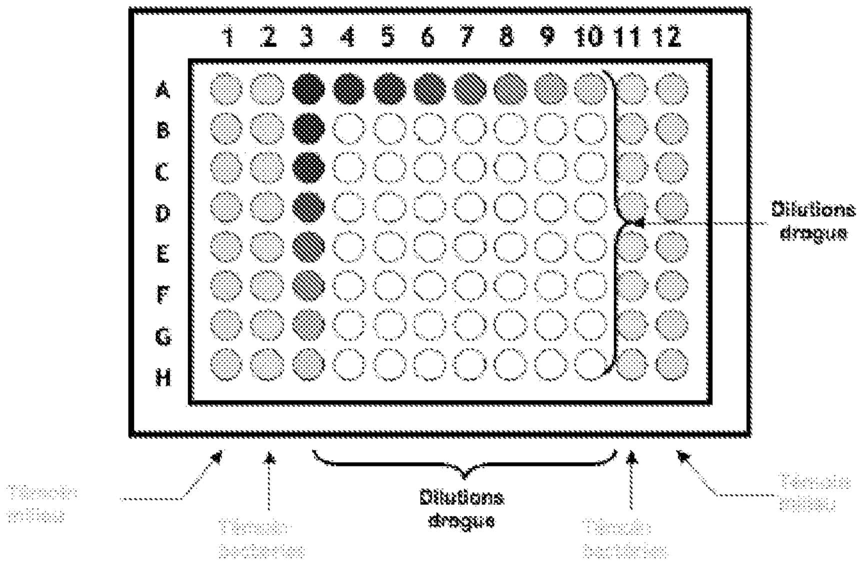


Figure 2A

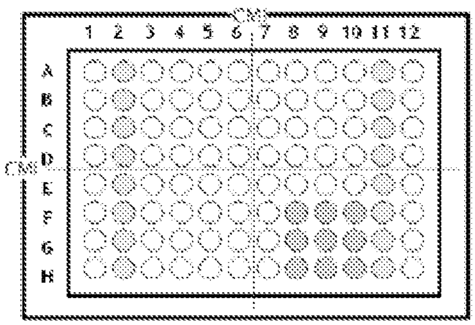


Figure 2B

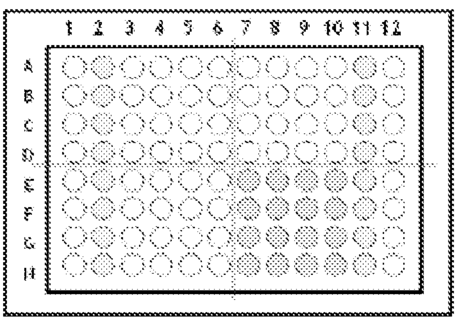

Figure 2C

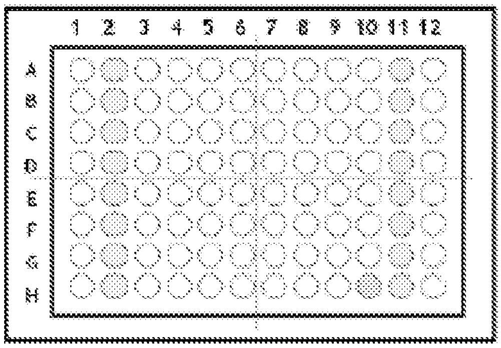


Figure 2D

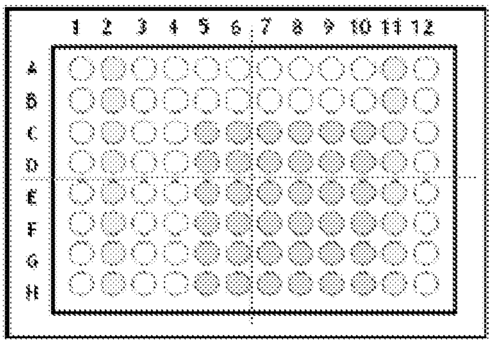

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **BOUCHER et al.** *CID,* 2009
- **GRARE et al.** *J. Antimicrob. Chemother.,* 2007, vol. 60, 575-581
- **GRARE et al.** *Clin. Microbiol. Infect.,* 2010, vol. 16, 432-438
- **GRARE et al.** *Pathologie Biologie,* 2010, vol. 58, 46-51
- **MOURER et al.** *Bioorganic & Médicinal Chemistry Letter,* 2006, vol. 16, 2960-2963
- **BERGE et al.** Journal of Pharmaceutical Science. *Pharmaceutical Salts,* 1977, vol. 66, 1-19